# EUROPEAN PATENT APPLICATION

(11) **EP 4 394 033 A1**
(43) Date of publication of application: **03.07.2024**
(21) Application number: 22860553.1
(22) Date of filing: 24.08.2022
(51) Int. Cl.: C12N 5/10, C12N 15/113, C12N 15/62, A61K 39/00, A61P 35/00

(54) **T CELL PRODUCT AND USE THEREOF**

(30) Priority: 24.08.2021 CN 202110976183
(71) Applicant: Cells & Genes Biotech (Shanghai) Co., Ltd, Shanghai 201203 (CN)
(72) Inventor: LI, Linhong, Shanghai 201203 (CN); ZHU, Xiangyang, Shanghai 201203 (CN); LIU, Wuqing, Shanghai 201203 (CN); YU, Shijun, Shanghai 201203 (CN); XIN, Pengcheng, Shanghai 201203 (CN); WANG, Li, Shanghai 201203 (CN); YANG, Xingxing, Shanghai 201203 (CN); ZHAN, Yifan, Shanghai 201203 (CN); WEI, Xiaoyue, Shanghai 201203 (CN)
(74) Representative: Santoro, Sofia
(86) International application number: PCT/CN2022/114587
(87) International publication number: WO 2023/025207

(57) **Abstract**

Provided are a T cell product for administration to a subject and a use thereof. The product comprises at least one allogeneic T cell, the allogeneic T cell expressing at least one MHC molecule identified as an exogenous source by at least one T cell of the subject. The T cell product can widen the range of a donor and activate an immune response of the subject. Further provided is a use of the T cell product in the preparation of a drug for treating tumors.

## Description

### Technical Field

The present application relates to the field of biomedicine, in particular to T cell product and use thereof.

### Background of the Invention

The main design idea of current universal CART cells (UCART) is to kill target cells. Under this idea, CART needs to survive *in vivo* for a long time. Therefore, in the practical application of UCART, two main problems need to be solved simultaneously: killing by UCART to the body of subjects (GVHD), and UCART is rejected by the body of subjects due to alloreaction. The current design of UCART mainly focuses on removing GVHD and alloreaction to avoid rejection by the body.But such a design may affect the efficiency of tumor treatment.

It is necessary to solve these two problems at the same time and effectively improve the tumor microenvironment, which brings challenges to the current UCART cell therapy.

Thus, there is an urgent need to obtain new "universal" T cell products that can effectively improve the therapeutic effect.

### Summary of the Invention

The present application provides a T cell product engineered to express at least one functional molecule and greatly reduce the possibility of GVHD for the administration to a subject and uses thereof. Meanwhile, the T cell product comprises at least one allogeneic T cell that can produce an allogeneic immune response. The cell expresses at least one MHC molecule, and the MHC molecule may be recognized as being exogenous by at least one T cell of the subject. In the present application, the T cell product has at least one of the following characteristics: (1) there is a reduced or even no requirement on the source of allogeneic T cell; in some cases, allogeneic T cell derived from different donors may be used after mixing; (2) there is a little or no requirement for the type of MHC molecule (e.g., human HLA genotype) expressed by allogeneic T cells; (3) the graft-versus-host disease caused by allogeneic T cell in the subject is reduced; (4) the specific retention of allogeneic T cell can cause an alloreaction to effectively improve intratumoral immune response of the subject; (5) the specific retention of allogeneic T cell by mixing multiple donor sources can cause alloreactions to more effectively improve immune response (such as TH-1 immune response) of the subject; (6) one or more desired proteins may be further expressed (e.g., after transfection using non-viral methods); (7) effectively improve the tumor microenvironment in the subject; (8) intratumoral injection may be performed; (9) can significantly reduce the preparation cost. The present application also provides uses of the T cell product in preparing pharmaceutical compositions, preparing a medicament for treating tumors, and/or improving tumor microenvironment.

In one aspect, the present application provides a T cell product for the administration to a subject, wherein the product comprises at least one allogeneic T cell that expresses at least one MHC molecule recognized as being exogenous by at least one T cell of the subject, and an alloreaction is caused after recognization of the MHC molecule by at least one TCR of the subject.

In some embodiments, the at least one allogeneic T cell is not subjected to an operation for reducing or eliminating immune rejection.

In some embodiments, the operation for reducing or eliminating immune rejection comprises knocking out CD52 gene of an allogeneic T cell; and/or changing HLA-I molecule of an allogeneic T cell.

In some embodiments, in the allogeneic T cell, a molecule capable of causing an alloreaction in a subject is not modified.

In some embodiments, the molecule capable of causing an alloreaction in a subject comprises a MHC molecule of the allogeneic T cell.

In some embodiments, the subject comprises a patient with a tumor.

In some embodiments, the tumor comprises a solid tumor and/or non-solid tumor.

In some embodiments, the allogeneic T cell is derived from a source different from the subject.

In some embodiments, the allogeneic T cell is derived from two or more donors that are different from the subject.

In some embodiments, the allogeneic T cell expresses a MHC molecule that is recognized as being exogenous by at least one T cell of the subject.

In some embodiments, the allogeneic T cell expresses two or more MHC molecules that are recognized as being exogenous by at least one T cell of the subject.

In some embodiments, the MHC molecule comprises MHC I and/or MHC II.

In some embodiments, the allogeneic T cell comprises helper T cell (Th) and/or killer T cell (CTL).

In some embodiments, the recognition of the MHC molecule by at least one TCR of the subject promotes a TH-1 immune response in the subject.

In some embodiments, the product has the ability to reduce the risk of causing graft-versus-host disease (GVHD) in the subject by the allogeneic T cell.

In some embodiments, compared with an allogeneic T cell without an engineering modification, the allogeneic T cell with an engineering modification reduces the risk of graft-versus-host disease (GVHD) in the subject by the allogeneic T cell.

In some embodiments, the engineering modification comprises the following step: down-regulating the expression and/or activity of a CD3-related gene, TRAC gene and/or TRBC gene in the allogeneic T cell.

In some embodiments, compared with the allogeneic T cell without an engineering modification, the expression and/or activity of a CD3-related gene, TRAC gene and/or TRBC gene is downregulated in the allogeneic T cell with an engineering modification.

In some embodiments, the engineering modification comprises gene mutation and/or gene silencing.

In some embodiments, the engineering modification comprises administering to the allogeneic T cell one or more substances selected from the group consisting of: an antisense RNA, siRNA, shRNA, CRISPR/Cas system, RNA editing system, RNA-guided endonuclease, zinc finger protease, Mega-TAL nuclease, TALENs, Sleeping beauty transposon system and Meganucleases.

In some embodiments, the engineering modification comprises administering a CRISPR/Cas system to the allogeneic T cell.

In some embodiments, the engineering modification comprises administering to the allogeneic T cell a sgRNA targeting the exon region of the CD3-related gene, TRAC gene and/or TRBC gene.

In some embodiments, at least one of the allogeneic T cell with modification expresses an antibody or antigen-binding fragment thereof, a chimeric antigen receptor (CAR), a cytokine and/or a chemokine.

In some embodiments, at least one of the allogeneic T cell with modification comprises a nucleic acid encoding an antibody or antigen-binding fragment thereof, a chimeric antigen receptor (CAR), a cytokine, and/or chemokine.

In some embodiments, the modification comprises the transfection of the nucleic acid into the allogeneic T cell.

In some embodiments, the transfection comprises the viral transfection and/or non-viral transfection.

In some embodiments, a plasmid is used for the non-viral transfection.

In some embodiments, the plasmid is a circular plasmid or a supercoiled plasmid.

In some embodiments, the plasmid comprises a nucleic acid encoding an antibody or an antigen-binding fragment thereof, a chimeric antigen receptor (CAR), a cytokine and/or a chemokine.

In some embodiments, the nucleic acid comprises a ssDNA and/or dsDNA.

In some embodiments, the plasmid comprises a plasmid produced by bacteria.

In some embodiments, the plasmid comprises a Nano plasmid and/or minicircle DNA vector.

In some embodiments, the transfection comprises the stable transfection and/or transient transfection.

In some embodiments, the transient transfection comprises the electroporation.

In some embodiments, the transient transfection comprises the mRNA transfection.

In some embodiments, the stable transfection comprises the knock-in by using a transposon system and/or using gene editing method.

In some embodiments, the gene editing method is selected from the group consisting of: a CRISPR/Cas system, RNA editing system ADAR, RNA-guided endonucleases, zinc finger nucleases, Mega-TAL nucleases, TALENs, and Meganucleases.

In some embodiments, the transposon system comprises a Sleeping beauty transposon system.

In some embodiments, the plasmid is used as a donor plasmid in a knock-in process via gene editing.

In some embodiments, in a transfection mixture compriseing the plasmid, the content of a nucleic acid molecule or fragment thereof derived from a microbial genome is less than about 10% (w/w) of the total content of nucleic acid molecules in the transfection mixture.

In some embodiments, in the transfection mixture comprising the plasmid, the content of a nucleic acid molecule or fragment thereof derived from a microbial genome is less than about 2% (w/w) of the total content of nucleic acid molecules in the transfection mixture.

In some embodiments, in the transfection mixture compriseing the plasmid, in the transfection mixture comprising the plasmid, the content of a nucleic acid molecule or fragment thereof derived from a microbial genome is less than about 5‰ (w/w)of the total content of nucleic acid molecules in the transfection mixture.

In some embodiments, in the transfection mixture comprising the plasmid, the content of a nucleic acid molecule or fragment thereof derived from a microbial genome is less than about 1%o (w/w) of the total content of nucleic acid molecules in the transfection mixture.

In some embodiments, in the transfection mixture comprising the plasmid, the content of a nucleic acid molecule or fragment thereof with a size of at least about 48 kb is less than about 10% (w/w) of the total content of nucleic acid molecules in the transfection mixture.

In some embodiments, in the transfection mixture comprising the plasmid, the content of a nucleic acid molecule or fragment thereof with a size of at least about 48 kb is less than about 2% (w/w) of the total content of nucleic acid molecules in the transfection mixture.

In some embodiments, in the transfection mixture comprising the plasmid, the content of a nucleic acid molecule or fragment thereof with a size of at least about 48 kb is less than about 5‰ (w/w) of the total content of nucleic acid molecules in the transfection mixture.

In some embodiments, in the transfection mixture comprising the plasmid, the content of a nucleic acid molecule or fragment thereof with a size of at least about 48 kb is less than about 1‰ (w/w) of the total content of nucleic acid molecules in the transfection mixture.

In some embodiments, the size of the nucleic acid molecule or a fragment thereof with a size of at least about 48 kb is at least about 1 Mb.

In some embodiments, the size of the nucleic acid molecule or a fragment thereof with a size of at least about 48 kb is at least about 10 Mb.

In some embodiments, the nucleic acid molecule or a fragment thereof with a size of at least about 48 kb is derived from a microorganism.

In some embodiments, the microorganism is selected from the group consisting of: bacteria, fungi, actinomycetes, mycoplasmas, chlamydiae, rickettsiae, and spirochetes.

In some embodiments, the microorganism comprises Gram-negative bacteria.

In some embodiments, the microorganism comprises *Escherichia coli.*

In some embodiments, the plasmid is treated with deoxyribonuclease (DNase).

In some embodiments, the DNase is capable of non-specifically cleaving linear DNA.

In some embodiments, the DNase is an exonuclease.

In some embodiments, the treatment comprises contacting the transfection mixture with the DNase in the presence of Mg²⁺ and Ca²⁺.

In some embodiments, after the treatment by the DNase, the content of a nucleic acid molecule or fragment thereof derived from a microbial genome in the transfection mixture comprising the plasmid is less than about 10% (w/w) of the total content of nucleic acid molecules in the transfection mixture.

In some embodiments, after the treatment by the DNase, the content of a nucleic acid molecule or fragment thereof with a size of at least about 48 kb in the transfection mixture comprising the plasmid is less than about 10% (w/w) of the total content of nucleic acid molecules in the transfection mixture.

In some embodiments, the antibody or an antigen-binding fragment thereof comprises a bispecific antibody.

In some embodiments, the antibody or an antigen-binding fragment thereof comprises a bispecific T cell engager (BiTE).

In some embodiments, the CAR comprises an antigen-binding domain, hinge region, transmembrane domain, costimulatory domain and signaling domain.

In some embodiments, the antigen-binding domain specifically binds one or more tumor-associated antigens.

In some embodiments, the antigen binding domain specifically binds GPC3 and CD73.

In some embodiments, the antigen binding domain specifically binds GPC3 and CD 147.

In some embodiments, the antigen binding domain specifically binds a tumor associated antigen (TAA).

In some embodiments, the antigen-binding domain is selected from the group consisting of: a monoclonal antibody, polyclonal antibody, human antibody, humanized antibody, single domain antibody, nano antibody, and antigen-binding fragment thereof.

In some embodiments, the hinge region comprises a polypeptide from a protein selected from the group consisting of: CD28, HLA-A, HLA-B, HLA-C, HLA-G, HLA-DQ, HLA-DP, HLA-DR, CD8, Fc, IgG, IgD, 4-1BB, CD4, CD27, CD7 and PD1.

In some embodiments, the transmembrane domain comprises a polypeptide from a protein selected from the group consisting of: CD27, CD7, PD1, TRAC, TRBC, CD28, CD3e, CD45, CD4, CD5, CD8, CD9, CD16, CD22, CD33, CD37, CD64, CD80, CD86, CD134, CD137 and CD154.

In some embodiments, the costimulatory domain comprises a polypeptide from a protein selected from the group consisting of: CD28, CD137, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, and ligand of CD83.

In some embodiments, the signaling domain comprises a polypeptide from a protein selected from the group consisting of: CD3ζ, FcRy (FCER1G), FcyRIIa, FcRβ (FcεR1b), CD3γ, CD3δ, CD3ε, CD79a, CD79b, DAP10, DAP12, Igα receptor, Igβ receptor, BLV gp30 (bovine leukemia virus gp30), EBV (Ep-stein-Barr virus) LMP2A, Simian Immunodeficiency Virus PBj14 Nef and the immunoreceptor tyrosine activation motif (ITAM).

In some embodiments, the cytokine is selected from the group consisting of: IL-1, IL-7, IL-12, IL-13, IL-15, IL-16, IL-17A, IL-17F, IL -18, IL-21, IL-23, TNFα, CXCL1, CD38, CD40, CD69, IgG, IP-10, L-17A, MCP-1 and PGE2.

In some embodiments, the chemokine is selected from the group consisting of: CCL1-CCL28 and CXCL1-CXCL17.

In some embodiments, after transfection, at least one of the allogeneic T cell comprises at least one plasmid comprising the nucleic acid.

In some embodiments, at least one of the allogeneic T cell expresses a chimeric antigen receptor (CAR), or expresses a bispecific T cell adapter (BiTE).

In some embodiments, at least one of the allogeneic T cell expresses two or more proteins.

In some embodiments, the gene encoding the CAR comprises the sequence shown in SEQ ID NO. 2 or SEQ ID NO: 11 or 12.

In some embodiments, the CAR comprises the amino acid sequence shown in SEQ ID NO. 10.

In some embodiments, at least two of the allogeneic T cells expressing different chimeric antigen receptor (CAR) and/or cytokine are mixed proportionally.

In some embodiments, the T cell product further comprises an autologous T cell of the subject.

In some embodiments, the T cell product further comprises one or more other modified T cells that have a reduced ability to cause an alloreaction in the body compared with the corresponding unmodified T cells.

In some embodiments, the other modified T cells have reduced expression and/or activity of MHC molecules compared with corresponding unmodified T cells.

In another aspect, the present application provides a pharmaceutical composition comprising the T cell product of the present application and a pharmaceutically acceptable excipient.

In some embodiments, the excipient is suitable for injection.

In some embodiments, the excipient is suitable for intratumoral injection.

In another aspect, the present application provides a kit comprising the T cell product of the present application and an injection equipment.

In another aspect, the present application provides use of the T cell product of the present application, the pharmaceutical composition of the present application and/or the kit of the present application in the manufacture of a medicament for the prevention, alleviation, treatment of a tumor.

In some embodiments, the tumor comprises a solid tumor.

In some embodiments, the medicament is formulated for injection.

In some embodiments, the medicament is formulated to be suitable for intratumoral injection.

In another aspect, the present application provides a method for improving tumor microenvironment, comprising the following step: administering to a subject the T cell product of the present application, the pharmaceutical composition of the present application, and/or the kit of the present application.

In another aspect, the present application provides a method for preventing, alleviating, and treating tumor, comprising the following step: administering to a subject the T cell product of the present application, the pharmaceutical composition of present application, and/or the kit of the present application.

In some embodiments, the administration comprises injection.

In some embodiments, the administration comprises intratumoral injection.

In some embodiments, the administration comprises the intratumoral injection for one or more times.

In another aspect, the present application provides a T cell product of the present application, a pharmaceutical composition o the present application and/or a kit of the present application for use in prevention, alleviation and treatment of a tumor.

Those skilled in the art will readily appreciate other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the descriptions in the drawings and specifications of the present application are illustrative only and not restrictive.

### Brief Description of the Drawings

The specific features of the invention involved in the present application are as shown in the appended claims. The features and advantages of the invention involved in the present application may be better understood by referring to the detailed description of the exemplary embodiments and the drawings. Brief descriptions of the drawings are as follows:
Figures 1A-1B show that T cells derived from different individuals can cause different levels of alloreactions with a specific allogeneic PBMC. However, the allogeneic T cells of the present application mixed from different donor sources will ensure a strong alloreaction with the specific allogeneic PBMC.
Figure 2 shows that the allogeneic T cells of the present application mixed from different donor sources can cause an alloreaction with an allogeneic PBMC.
Figure 3 shows that allogeneic T cells of the present application derived from one or more donors are capable of expressing CAR molecules.
Figure 4 shows that the T cells of the present application can express CAR molecules for a long period.
Figures 5A-5B show that the allogeneic T cell of the present application can express two or more molecules simultaneously.
Figure 6 shows that the engineered and/or modified allogeneic T cell of the present application can express multiple molecules simultaneously, and after killing tumor cells, release other cytokines in addition to the specifically expressed molecules.
Figures 7A-7F show that the engineered and/or modified allogeneic T cell of the present application can specifically kill tumor cells.
Figure 8 shows that the abilities of allogeneic T cells of the present application derived from one or more donors to kill tumor cells are equivalent.
Figures 9A-9B show the supernatant after killing tumor cells by the allogeneic T cell of the present application derived from one or more donors has a comparable ability to recruit immune cells.
Figures 10A-10B show the supernatant after killing tumor cells by the allogeneic T cell of the present application derived from one or more donors has the ability to activate immune cells (9A), and the activated PBMC has the ability to kill K562 cells.
Figures 11A-11B show the ability of engineered and/or modified allogeneic T cells of the present application derived from multiple donors to express molecules.
Figure 12 shows that a mixture of allogeneic T cells of the present application derived from multiple donors that have been chemically modified and/or modified can effectively kill tumor cells.
Figure 13 shows that the supernatant of a mixture of engineered and/or modified allogeneic T cell of the present application derived from multiple donors can assist PBMC to effectively kill tumor cells.
Figure 14 shows that the supernatant of a mixture of engineered and/or modified allogeneic T cell of the present application derived from multiple donors after killing target cells can effectively activate T cells and/or NK cells.
Figures 15A-15C show that the supernatant of the engineered and/or modified allogeneic T cell of the present application after killing tumor cells can activate T/NK cells.
Figure 16 shows that the engineered and/or modified allogeneic T cell of the present application may be activated and expanded by target cells multiple times, revived and/or cryopreserved.
Figure 17 shows that the engineered and/or modified allogeneic T cell of the present application can effectively kill tumor cells.
Figure 18 shows that the cell supernatant of the engineered and/or modified allogeneic T cell of the present application after killing tumor cells has the ability of By-Stand activation of T cells in PBMCs.
Figure 19 shows that after multiple activation and expansion of the engineered and/or modified allogeneic T cell of the present application, the cell supernatant after killing tumor cells has the ability of By-Stand activation of NK cells in PBMCs.
Figure 20 shows that the engineered and/or modified allogeneic T cell of the present application can effectively reduce tumor volume *in vivo.*
Figure 21 shows an increase in the amount of XCL-1 expressed in the supernatant of a mixture of engineered and/or modified allogeneic T cell of the present application derived from multiple donors after killing target cells.
Figure 22 shows that the supernatant after killing target cells by a mixture of engineered and/or modified allogeneic T cell of the present application derived from multiple donors can effectively recruit cDC 1 cells.
Figure 23 shows that the supernatant after killing target cells by a mixture of engineered and/or modified allogeneic T cell of the present application derived from multiple donors can effectively recruit T/NK cells.
Figure 24A shows that the cell supernatant of the engineered and/or modified allogeneic T cell of the present application derived from multiple donors after killing target cells has the ability of By-Stand activation of NK cells in PBMCs.
Figure 24B shows that the cell supernatant of the engineered and/or modified allogeneic T cell of the present application derived from multiple donors after killing target cells has the ability of By-Stand activation of T cells in PBMCs.
Figure 25 shows PBMCs activated by the cell supernatant of the engineered and/or modified allogeneic T cell of the present application derived from multiple donors after killing target cells can significantly improve the efficiency of killing K562 cells.
Figure 26 shows that the engineered and/or modified allogeneic T cell of the present application can significantly inhibit the growth of tumors.
Figure 27 shows that the engineered and/or modified allogeneic T cell of the present application can effectively kill tumors *in vivo* and reduce tumor volume through multiple intratumoral injections.

### Detailed description

The following specific examples illustrate the embodiments of the present invention, and those skilled in the art can easily understand other advantages and effects of the present invention from the content disclosed in this specification.

### Terms

In the present application, the term "T cell product" generally refers to a therapeutic product compriseing T cells that may be administered to a subject. In the present application, the T cell product may be used for cell therapy. For example, the T cell product may comprise T cells capable of expressing therapeutically effective proteins (e.g., chimeric antigen receptors, antibodies or antigen-binding proteins thereof, cytokines, and/or chemokines). In the present application, the T cell product may be used to treat tumors. In some cases, the T cell product may be administered to the subject by administration means such as injection (e.g., intratumoral injection). In the present application, the T cell product may be personalized, for example, designed to meet the actual needs of the subject in need.

In the present application, the term "T cell" generally refers to thymus-derived cells. The T cells can participate in various cell-mediated immune responses. In the present application, the T cells may comprise thymocytes, naive T lymphocytes, immature T lymphocytes, mature T lymphocytes, resting T lymphocytes or activated T lymphocytes. In some cases, markers may be used to classify the T cells. For example, the T cells may comprise helper T cells (CD4⁺ T cells), killer T cells (CTL; CD8⁺ T cells), CD4⁺CD8⁺ T cells, and/or CD4⁻CD8⁻ T cells. For example, the T cells may express one or more of the following markers: CD3, CD4, CD8, CD27, CD28, CD45RA, CD45RO, CD62L, CD127, CD197, and HLA-DR.

In the present application, the term "helper T cells (Th)" generally refers to effector T cells that promote the activation and function of other B and T lymphocytes and/or macrophages. In the present application, the helper T cells may be CD4⁺ T cells. When activated, the helper T cells can promote the body's adaptive immune response. In some cases, the helper T cells can participate in antigen-specific immune responses. The helper T cells may be further differentiated into different subpopulations, for example, secreting cytokines and/or chemokines through different transcription factors. For example, different immune cells may be recruited and/or different immune response mechanisms may be coordinated. The helper T cells may comprise Th1 cells, Th2 cells, Th17 cells and/or regulatory T cells (Treg).

In the present application, the term "TH-1 immune response" generally refers to an immune response in which Th1 cells participate. Th1 cells can recognize antigens presented by major histocompatibility complex (MHC) class I or class II molecules. Th1 cells may be involved in the recognition and/or clearance of intracellular pathogens (e.g., pathogens present within phagocytic vesicles in macrophages). In the present application, the TH-1 immune response may comprise activating the function (such as killing and lytic function) of other immune cells (such as macrophages, B cells, killer T cells) through Th1 cells (for example, through the interaction of CD40L on the surface of Th1 cells and CD40 expressed on the surface of macrophages, B cells or dendritic cells) and/or substances secreted by them (such as IFNy). In the present application, the TH-1 immune response may also be involved in tumor killing. For example, killer T cells may be activated to target and kill tumor cells; for example, the survival ability and/or memory ability of killer T cells may be enhanced.

In the present application, the term "killer T cells (CTL)" generally refers to CD8⁺ T cells. The killer T cells participate in adaptive immune responses and can play a role in immune defense against pathogens within cells and in killing tumors. The surface of the killer T cells can express CD8⁺ co-receptor; the killer T cells can perform an immune response to the antigen presented by MHC class I molecules. For example, the killer T cells can bind to Fas receptors and/or Fas ligands expressed on lymphocytes and/or infected target cells to induce apoptosis of the latter. For example, the killer T cells can produce a variety of cytokines (e.g., IFNy, TNFα). For example, the killer T cells in the tumor microenvironment can kill tumor cells through the cytotoxicity of cytokines. In the present application, the killer T cells may comprise Tc1, Tc2, Tc9, Tc17 and/or CD8⁺ Tregs.

In the present application, the term "allogeneic T cell" generally refers to T cells from different sources in a subject. For example, the allogeneic T cell may be derived from a different donor than the subject. In some cases, the allogeneic T cell may be derived from more than two (e.g., 2, 3, 4 or more) donors different from the subject. In the present application, the allogeneic T cell may express MHC molecules. In some cases, at least one of the MHC molecules may be recognized by at least one T cell of the subject as "exogenous (e.g., may be considered not to be produced by the body of the subject) MHC molecules. In the present application, the allogeneic T cell may be T cells obtained directly from the donor. In some cases, the allogeneic T cell may be modified. For example, the allogeneic T cell may be engineered to reduce the risk of causing graft-versus-host disease (GVHD) in the subject. For example, the allogeneic T cell may be modified to express at least one protein required for treating the disease of the subject.

In the present application, the term "subject" generally refers to mammals, such as humans, non-human primates (e.g., apes, gibbons, gorillas, chimpanzees, orangutans, macaques), domestic animals (e.g., dogs and cats), farm animals (e.g., poultry such as chickens and ducks, horses, cattle, goats, sheep, pigs) and laboratory animals (e.g., mice, rats, rabbits, guinea pigs). Human subjects may comprise fetal, neonatal, infant, adolescent, and adult subjects. Subjects may comprise animal disease models, such as mouse models, and other animal models known to those skilled in the art. In the present application, a subject in need thereof may comprise a patient, such as a tumor patient.

In the present application, the term "exogenous" generally refers to any substance introduced from or produced outside an organism, cell, tissue or system. For example, at least one MHC molecule expressed by the allogeneic T cell may be recognized by a T cell of the subject as exogenous to the subject (e.g., MHC molecules produced external to the subject).

In the present application, the term "MHC molecule" generally refers to the major histocompatibility complex (MHC), which is a general name for a group of genes encoding major histocompatibility antigens in animals. Human MHC can also be called HLA (human leukocyte antigen, HLA) complex. Due to the polygenic nature of MHC, MHC may be divided into genes of MHC class I, MHC class II, and MHC class III based on the structure, tissue distribution, and functional differences of the molecules they encode, and encode MHC class I molecules, MHC class II molecules, and MHC III genes respectively. In the present application, the MHC molecules may also comprise those proteins involved in delivering the antigen to MHC for presentation. MHC class I molecules may be distributed on the surface of the nucleated cells. MHC class I molecules can participate in the process of antigen presentation to CD8⁺ T cells. MHC class II molecules may be distributed on the surface of antigen-presenting cells such as B cells, monocytes-macrophages, and dendritic cells. MHC class II molecules can present processed antigen fragments to CD4⁺ T cells. MHC class II molecules may cause graft rejection.

In the present application, the term "TCR" generally refers to a specific receptor on the surface of T cells. The T cell receptor is a heterodimer, which may be composed of two different subunits. Most T cell receptors (for example, more than 95%, more than 96%, more than 97%, etc.) are composed of α subunit and β subunit, and each peptide chain may be divided into variable regions (V regions), constant region (C region), transmembrane region and cytoplasmic region. In the present application, the TCR may comprise any TCR or functional fragment, such as specific antigenic peptide bound in an MHC molecule, i.e., antigen-binding portion of the TCR of an MHC-peptide complex. The "antigen-binding portion" or "antigen-binding fragment" of a TCR, used interchangeably, refers to a molecule that comprises portion of the domain of the TCR but binds the full-length TCR-bound antigen (e.g., an MHC-peptide complex). In some cases, the antigen-binding portion may comprise variable domains of the TCR, such as the variable alpha chain and the variable beta chain of the TCR, which are sufficient to form a binding site for binding to a specific MHC-peptide complex., as generally speaking, each chain comprises three complementarity determining regions.

In the present application, the term "donor" generally refers to the species from which the graft is derived. For example, the donor may be in a dead state, and the donor may be in a living state. In the present application, the donor may be in a healthy state. For example, the donor may not have a tumor. The donor may be mammals such as humans, non-human primates (e.g., ape, gibbon, gorilla, chimpanzee, orangutan, macaque), domestic animals (e.g., dog and cat), farm animals (e.g., poultry such as chicken and ducks, horses, cattle, goats, sheep, pigs) and experimental animals (e.g., mice, rats, rabbits, guinea pigs). In the present application, the subject and the donor may both be of the same species, for example, both human. For example, the donor may also comprise a donor that is genetically different from the subject, a donor that is cross-matched incompatible with the subject, and/or a donor that is cross-matched compatiblewith the subject.

In the present application, the term "alloreaction" generally refers to an immune response produced by organs, tissues and/ or cells derived from a donor. The alloreaction may comprise stimulations and responses of the immune system in response to alloantigens or "allogeneic antigens" or cells expressing different HLA haplotypes. In the present application, the alloreaction may comprise the stimulation and response to the subject caused by the organs, tissues and/or cells derived from the donor; the alloreaction may also comprise the subject's stimulation and response to the organ, tissue and/or cell derived from the donor. For example, the alloreaction may comprise graft versus host disease (GVHD). For example, the alloreaction may comprise graft rejection.

As used herein, the term "graft-versus-host disease (GVHD)" generally refers to an acute or chronic immune response in a subject due to exogenous (e.g., donor-derived) organs, tissues, and/or cells. For example, organs, tissues and/or cells obtained from other individuals of the same species (even HLA matched in some cases) can subject a subject to graft versus host disease. The GVHD may produce symptoms on the intestinal epithelium and/or liver of the subject. In some cases, the GVHD can cause an inflammatory response in the skin, liver, stomach and/or intestines. In some cases, the GVHD is chronic, and common symptoms can comprise rashes and/or mouth ulcers. The GVHD is currently largely incurable.

In the present application, the term "intratumoral injection" generally refers to administration directly into the tumor cell mass and/or the tumor microenvironment. In the present application, the tumor microenvironment may comprise a tumorigenic environment, which may create a structural and/or functional environment for tumor processes to survive, expand or spread. The tumor microenvironment may be composed of cells, molecules, fibroblasts, extracellular matrix, and blood vessels that surround and feed the tumor cell or cells that form the tumor. Examples of cells or tissues in the tumor microenvironment comprise, but are not limited to, tumor vasculature, tumor infiltrating lymphocytes, fibroblast reticular cells, endothelial progenitor cells (EPCs), cancer-associated fibroblasts, pericytes, other stromal cells, extracellular matrix (ECM), dendritic cells, antigen-presenting cells, T cells, regulatory T cells, macrophages, neutrophils and other immune cells close to the tumor. Examples of cellular functions that influence the tumor microenvironment may comprise, but are not limited to, production of cytokine and/or chemokine, responses to cytokines, antigen processing and presentation of peptide antigens, regulation of leukocyte chemotaxis and migration, regulation of gene expression, complement activation, regulation of signaling pathways, cell-mediated cytotoxicity, cell-mediated immunity, humoral immune responses, and other innate or adaptive immune responses. Measure the effects of modulating the function of these cells.

In the present application, the term "pharmaceutical composition" generally refers to a composition suitable for administration to a patient, such as a human patient. Pharmaceutical compositions of the present application comprise a therapeutically effective amount of one or more antibody constructs of the present application. Pharmaceutical compositions may also comprise suitable preparations containing one or more (pharmaceutically effective) carriers, stabilizers, excipients, diluents, solubilizers, surfactants, emulsifiers, preservatives and/or adjuvants. For example, acceptable ingredients of the composition are not toxic to the subject at the doses and concentrations used. The pharmaceutical compositions of the present application comprise, but are not limited to, liquid, frozen and lyophilized compositions.

In the present application, the term "tumor" generally refers to premalignant and malignant cancers. The tumors may comprise primary tumors (e.g., those whose cells have not migrated to a site in the subject other than the site of the original tumor) and secondary tumors (e.g., those tumors that are caused by metastasis, tumor cells migrating to a secondary site different from the original tumor site), recurrent cancers and refractory cancers. The tumors may comprise solid tumors and non-solid tumors.

In the present application, the term "deoxyribonuclease (DNase)" generally refers to an enzyme capable of cleaving phosphodiester bonds on the DNA backbone. The DNase may be a type of nuclease. The DNase can digest double-stranded DNA into deoxynucleotides (for example, under weakly alkaline conditions). For example, the DNase may be substantially inactive (e.g., unable to cleave) circular or supercoiled nucleic acid molecules or fragments thereof (e.g., closed-circular double-stranded DNA and/or supercoiled DNA). For example, the DNase can cleave linear double-stranded DNA. For example, the DNase may be a DNA exonuclease. For example, the DNase may be exonuclease V. For example, the DNase may be ATP-Dependent DNase, such as Plasmid-Safe^{™} ATP-Dependent DNase.

In the present application, the term "transient transfection" generally refers to a transfection method in which the exogenous gene transfected into the cell is not integrated into the genome of the cell. Transient transfection can achieve rapid expression of exogenous genes in a short period (e.g., at least about 1 day, at least about 2 days). The exogenous gene transfected into the cell by transient transfection may be gradually lost as the cell grows and/or divides. The transient transfection may have advantages selected from the following group: easy operation, short experimental cycle, high expression efficiency, safety, and no need for gene screening. The operation steps of the transient transfection may be known to those skilled in the art. For example, the transient transfection may be achieved via liposome mediated transfection. For example, the transient transfection may comprise electroporation transfection (e.g., electroporation). The transient transfection can use transfection reagents, such as FuGENE6.

In the present application, the term "stable transfection" generally refers to that exogenous nucleic acid molecules are introduced and intergrated into the genome of the transfected cells. For example, the exogenous gene is intergrated into the genome of the transfected cells.

In the present application, the term "transfection mixture" generally refers to a mixture required for transfection. In the present application, the transfection mixture may comprise one or more materials to be introduced (e.g., polynucleotides). For example, the transfection mixture may comprise a nucleic acid molecule encoding an exogenous gene to be transfected. The transfection mixture may comprise a vector containing the nucleic acid molecule. For example, the transfection mixture may also comprise impurities (in some cases, the impurities may comprise nucleic acid molecule or fragment thereof). In the present application, the impurities may comprise nucleic acid molecule or fragment thereof derived from microorganisms. The impurities carried by the vector may include impurities carried by the vector (e.g., nucleic acid molecule or fragment thereof homologous or heterologous to the vector backbone).

In the present application, the term " total content of nucleic acid molecules" generally refers to the total mass of all substances with nucleotides in the transfection mixture. For example, the substances with nucleotides may comprise the nucleic acid molecule or fragment thereof and the materials.

In the present application, the term "microorganism" generally refers to eukaryotic and prokaryotic microbial species from the *Archaea, Bacteria,* and/or *Eucarya* domains. For example, the microorganisms may comprise bacteria, viruses, fungi, actinomycetes, rickettsiae, mycoplasmas, chlamydiae, and/or spirochetes. In the present application, the term bacteria generally refers to any type of prokaryote, including prokaryotes from all phyla within the prokaryote domain. The bacteria may comprise cocci, bacilli, spirilla, protoplasts, spheroplasts. The bacteria may comprise Gram-positive and Gram-negative bacteria. "Gram-negative" and "Gram-positive" refer to staining patterns using the Gram stain method, which is well known in the art (see, for example, Finegold and Martin, Diagnostic Microbiology, 6th edition, CV Mosby St. Louis, pages 13-15 (1982)).

In the present application, the term "nucleic acid molecule or fragment thereof derived from a microbial genome " generally refers to a nucleic acid molecule or fragments thereof, which are derived from the genome of the microorganism. Information about the genomes of microorganisms may be found in A genomic catalog of Earth's microbiomes, Nature Biotechnology (2020).

In the present application, the term "Gram-negative bacteria" generally refers to bacteria that do not retain the primary dye used in Gram staining but are stained by the counterstain. Therefore, Gram-negative bacteria usually appear red in the Gram staining method. The cell wall of the Gram-negative bacteria has a lower content of peptidoglycan and a higher content of lipids. For example, the cell wall of the Gram-negative bacteria may have a lipopolysaccharide layer. For example, the Gram-negative bacteria may comprise *Escherichia coli, Pseudomonas aeruginosa, Proteus species, Shigella species, Klebsiella pneumoniae, Brucella species, Haemophilus influenzae, Haemophilus parainfluenzae, Moraxella catarrhalis, Acinetobacter species, Yersinia species, Legionella pneumophila, Bordetella pertussis, Bordetella parapertussis, Shigella species, Pasteurella species, Vibrio cholerae, Bacillus parahaemolyticus,* and/or *Plesimonas shigelloides.*

In the present application, the term *"Escherichia coli"* generally refers to *Escherichia coli. Escherichia coli* belongs to the *Enterobacteriaceae* family, *Escherichia* genus.

In the present application, the term "plasmid" generally refers to a construct containing genetic material. The plasmid may be designed to deliver genetic material (e.g., one or more nucleic acid sequences) into cells. The plasmid may contain autonomously replicating sequences of single-stranded or double-stranded nucleic acids (e.g., DNA or RNA) from any source. The term "plasmid" may be used interchangeably with the term "vector" in the present application. The plasmid may have different conformations, such as linear plasmids, circular plasmids, or supercoiled plasmids. The linear plasmid may be a linear DNA molecule. The supercoiled plasmid may comprise two nucleic acid strands that maintain intact structures (e.g., covalently closed circular DNA, cccDNA), and exhibit a supercoiled conformation. The circular plasmid may have at least one nucleic acid strand maintaining a complete circular structure. The plasmid may be not integrated into the cell genome.

In the present application, the term "gene editing" generally refers to the manipulation of inserting, deleting, and/or replacing nucleic acids into the genome. The gene editing may be achieved through homology-directed repair (HDR), non-homologous end joining (NHEJ), or single-base changes. The gene editing can use gene editing tools familiar to those skilled in the art, such as the zinc finger nuclease system (ZFN), the TALEN system, and/or CRISPR technique.

In the present application, the term "gene editing knock-in" generally refers to a genetic engineering process (e.g., also called knock-in), which involves one-to-one replacement of DNA sequence information at a genetic locus or insertion of sequence information not found within the endogenous locus. The gene editing knock-in can utilize homologous recombination. For example, using homologous recombination, an exogenous functional gene (a gene not originally present in the genome or an inactivated gene) is transferred into a cell and undergoes homologous recombination with homologous sequences in the genome, allowing it to be inserted into the genome and expressed in the cell. For example, the gene editing knock-in may at least partially replace the cellular genome with an exogenous gene. The gene editing knock-in can use CRISPR technique to achieve targeted "knock-in".

In the present application, the term "transposon system" generally refers to a system comprising the ability to excise from a donor polynucleotide (e.g., plasmid) and integrate into a target (e.g., cellular genomic DNA). The polynucleotide may be integrated into the target by a transposase. The transposon system may comprise a transposase. The transposase may transpose and mediate the transposed functional nucleic acid-protein complex. The transposon system may comprise transposon ends. The transposon ends may be the nucleotide sequences necessary for forming a complex with the transposase or integrase. The transposon ends may be double-stranded DNA. In some cases, the transposon ends may form a functional nucleic acid-protein complex with the transposon in the transposition reaction.

In the present application, the term "nanoplasmid" generally refers to a nanoplasmid, i.e., one or more lipid-based nanocarriers, polymeric nanoparticles, metallic nanoparticles, surfactant-based emulsions, dendrimers, buckyballs, nanowires, virus-like particles, peptideor protein-based particles (e.g., albumin nanoparticles), and/or nanoparticles produced using compositions of nanomaterials (e.g., lipid-polymer nanoparticles). In some cases, the size of the nanoplasmid may be equal to or less than about 100 nanometers.

In the present application, the term "minicircle DNA vector" generally refers to a product of site-specific recombination of a parental plasmid (PP). The minicircle DNA vector is a supercoiled DNA molecule. Under the effect of recombinase, the parental plasmid is transformed into two circular DNAs, one containing a large amount of bacterial backbone sequences, called miniplasmid (MP); the other is a eukaryotic expression cassette containing only the target gene, i.e., minicircle (MC). The minicircle DNA vector may comprise a eukaryotic expression box.

In the present application, the term "antibody" generally refers to an immunoglobulin that is reactive to a specific protein, peptide, or fragments thereof. Antibodies may be from any class, including but not limited to IgG, IgA, IgM, IgD, and IgE, and antibodies from any subclass (e.g., IgG1, IgG2, IgG3, and IgG4). Antibodies may have heavy chain constant regions selected from, for example, IgG1, IgG2, IgG3, or IgG4. Antibodies may also have light chains selected from, for example, kappa (κ) or lambda (λ). The antibodies of the present application may be derived from any species.

In the present application, the term "antigen-binding fragment" generally refers to a portion of an antibody molecule that comprises amino acid residues that interact with the antigen and confer specificity and affinity of the antibody to the antigen. Examples of antigen-binding fragments may comprise, but are not limited to, Fab, Fab', F(ab)₂, Fv, F(ab')₂, scFv, di-scFv and/or dAb. In the present application, the term "Fab" generally refers to a fragment containing the heavy chain variable domain and the light chain variable domain, as well as the light chain constant domain and the first constant domain of the heavy chain (CH1); the term "Fab"' generally refers to a fragment that differs from Fab by the addition of a small number of residues (including one or more cysteines from the antibody hinge region) at the carboxy terminus of the heavy chain CH1 domain; the term "F(ab')₂" generally refers to a dimer of Fab', an antibody fragment containing two Fab fragments connected via disulfide bridges in the hinge region. The term "Fv" generally refers to the minimal antibody fragment containing the complete antigen recognition and binding site. In some cases, this fragment may consist of a tight non-covalent dimer composed of a heavy chain variable region and a light chain variable region; the term "dsFv" generally refers to a disulfide-stabilized Fv fragment, in which the bond between a single light chain variable region and a single heavy chain variable region is a disulfide bond. The term "dAb fragment" generally refers to an antibody fragment composed of a VH domain. In the present application, the term "scFv" generally refers to a monovalent molecule in which one heavy chain variable domain and one light chain variable domain of an antibody are covalently linked together via a flexible peptide linker; such scFv molecules may have the general structure: NH₂-VL-linker-VH-COOH or NH₂-VH-linker-VL-COOH.

In the present application, the term "chimeric antigen receptor" (CAR) generally refers to a fusion protein that comprises an extracellular domain capable of binding to an antigen and at least one intracellular domain. CAR is the core component of chimeric antigen receptor T cells (CAR-T), which may comprise an antigen-binding domain (e.g., tumor-specific antigen and/or tumor-associated antigen), a transmembrane domain, a co-stimulatory domain, and an intracellular signaling domain. In the present application, the CAR may be based on the antigen-specificity (e.g., CD19) of an antibody and the intracellular domain of a T cell receptor activation. Genetically modified T cells expressing CAR may specifically recognize and eliminate malignant cells expressing the target antigen. For descriptions of CAR and CAR-T cells, see for example Sadelain M, Brentjens R, Rivi 'ere I. The basic principles of chimeric antigen receptor design. Cancer Discov. 2013;3(4):388-398; Turtle CJ, Hudecek M, Jensen MC, Riddell SR. Engineered T cells for anti-cancer therapy. Curr Opin Immunol. 2012;24(5):633-639; Dotti G, Gottschalk S, Savoldo B, Brenner MK. Design and development of therapies using chimeric antigen receptor-expressing T cells. Immunol Rev. 2014;257(1):107-126; and WO2013154760, WO2016014789.

In the present application, the term "universal" generally refers to cells that solve the graft-versus-host disease (GVHD) problem of the present application. For example, the universal T cells (U-T cells) may not cause graft-versus-host disease (GVHD) in the subject. For example, the universal T cells (U-T cells) may be used in all subjects (or hosts). For example, the universal T cells (U-T cells) may be allogeneic T cell to the subject.

In the present application, the term "bispecific antibody" generally refers to an antibody having a variable region capable of recognizing more than one epitope on one or more antigens. Bispecific antibodies comprise, but are not limited to, full-length antibodies, antibodies with two or more VL and VH structural domains, antibody fragments, such as Fab, Fv, dsFv, scFv, bispecific antibodies, and covalently or non-covalently linked antibody fragments. In some cases, the bispecific antibody may recognize two different epitopes on the same or different antigens. In some cases, the bispecific antibody may recognize two different antigens.

In the present application, the term "antigen-binding domain" generally refers to a structural domain capable of binding to a target antigen. Antigen-binding domains may comprise specific antigen-binding antibodies and antigen receptors or fragments thereof, antibodies or their antigen-binding fragments. Antigen-binding domains may be domains capable of binding to tumor-associated antigens, comprising but not limited to: CD19, CD20, CD22, CD123, CD33/IL3Ra, CD138, CD33, BCMA, CS1, C-Met, EGFRvIII, CEA, Her2, GD2, MAG3, GPC3, and NY-ESO-1.

In the present application, the term "BiTE" generally refers to bispecific T cell engagers. The BiTE may be a single polypeptide chain molecule having two antigen-binding domains, one of which binds to a T cell antigen and the second to an antigen present on the surface of a target cell (see WO 2005/061547; Baeuerle, P et al. (2008) "BiTE®: A New Class Of Antibodies That Recruit T Cells Drugs of the Future 33:137-147; or Bargou et al. (2008) "Tumor Regression in Cancer Patients by Very Low Doses of a T Cell-Engaging Antibody," Science 321:974-977).

In the present application, the term "about" generally refers to a value or numeric range within 30%, 25%, 20%, 15%, 10%, 9%, 8%, 7%, 6%, 5%, 4%, 3%, 2%, 1%, 0.5%, or 0.05% of the specified value or range involved in the present application. In the present application, when the term "about" precedes the first value in a series of two or more values, it applies to each value in the series.

### Detailed Description of the Invention

### T cell products and allogeneic T cell

In one aspect, the present application provides a T cell product for administration to a subject (e.g., a subject in need) comprising at least one allogeneic T cell that expresses at least one MHC molecule recognized as exogenous by at least one T cell of the subject, wherein an alloreaction is caused after recognization of the MHC molecule by at least one TCR of the subject.

In the present application, the ability of the at least one allogeneic T cell to cause an alloreaction in the subject is not eliminated or reduced. For example, the T cell product may comprise at least one allogeneic T cell that retains the ability to cause an alloreaction in the subject.

In the present application, the at least one allogeneic T cell does not undergo operation for reducing or eliminating immune rejection.

In the present application, the operation of reducing or eliminating immune rejection may comprise knocking out CD52 gene of an allogeneic T cell. In the present application, the operation of reducing or eliminating immune rejection may comprise changing HLA-I molecules of an allogeneic T cell.

In the present application, in the allogeneic T cell, a molecule capable of causing an alloreaction of a subject is not modified.

In the present application, the molecule capable of causing an alloreaction in a subject may comprise the MHC molecule of the allogeneic T cell.

In the present application, the subject may comprise a patient with tumor. For example, the patient with tumor can use the T cell product of the present application to treat the tumor (for example, suppress the proliferation of tumor cells, and/or slow down the course of the tumor), and/or alleviate the symptoms of the tumor. For example, the T cell product of the present application may be used as a cell therapy product needed by the subject.

In the present application, the tumor may comprise solid tumors and/or non-solid tumors. For example, the tumor may comprise liver cancer.

In the present application, the T cell product may also comprise T cells derived from the subject's autologous source.

In the present application, the T cell of the subject may comprise effector T cells, naive T cells, memory T cells, helper T cells (Th) and/or killer T cells (CTL).

In the present application, the T cell of the subject may express TCR. For example, the T cell may express a TCR that may be involved in recognizing MHC molecules. In some cases, the T cell of the subject may have the ability to recognize MHC molecules.

In the present application, the allogeneic T cell may be derived from a source different from the subject. In the present application, the source may comprise different individuals derived from the same species. For example, the donor may be a healthy person. For example, the donor may be a person who does not suffer from a neoplastic disease. In some cases, the donor may not be related to the subject. In some cases, the donor may not be an HLA match to the subject. For example, the source may comprise a different individual derived from the same species as the subject.

In the present application, the allogeneic T cell may be derived from more than two donors different from the subject. For example, at least 2, at least 3, at least 4 or more different donors may be derived from the same species as the subject.

In the present application, the allogeneic T cell may express an MHC molecule that is recognized as exogenous by at least one T cell of the subject. In the present application, there may be no requirement for MHC (human HLA) matching of the allogeneic T cell in the T cell product. In some cases, the T cell product may have no requirements on the donor's MHC (human HLA) matching of the allogeneic T cell and/or the number of donors. The T cell products of the present application may expand the source of the donors.

In the present application, the allogeneic T cell may have a biomarker selected from the following group: up to about 0.5% (for example, may be up to about 0.4%, up to about 0.3%, up to about 0.2%, up to about 0. 1%, up to about 0.05% or less) CD3, about 50% to about 100% CD8 (for example, may be about 50% to about 95%, about 50% to about 90%, about 50% to about 85%, about 50% to about 80%, about 55% to about 100%, about 60% to about 100%, or about 55% to about 95%), and about 0% to about 40% CD4 (for example, may be about 0% to about 40%, about 0% to about 35%, about 0% to about 30%, about 0% to about 25%, about 5% to about 40%, or about 10% to about 40%). In the present application, the ratio may be a ratio range detected by flow cytometry. In the present application, the allogeneic T cell may not substantially express CD3. For example, in the allogeneic T cell, the ratio of cells that express CD3 may be up to about 0.5% (e.g., up to about 0.4%, up to about 0.3%, up to about 0.2%, up to about 0.1%, up to about 0.05%, or lower).

In the present application, the allogeneic T cell may express two or more MHC molecules recognized by T cells as exogenous (for example, they may be at least 2, at least 3, at least 4, at least 5, at least 6, at least 7, at least 8, at least 9, at least 10 or more) by at least one of the subjects (for example, it may be at least 1, at least 2, at least 3, at least 4, at least 5, at least 6, at least 7, at least 8, at least 9, at least 10 or more).

In the present application, the MHC molecules may comprise MHC I and/or MHC II.

In the present application, the allogeneic T cell may comprise effector T cells, naive T cells, memory T cells, helper T cells (Th) and/or killer T cells (CTL). In the present application, the allogeneic T cell may comprise helper T cells (Th) and/or killer T cells (CTL).

In the present application, at least one of the TCRs of the subject can cause an alloreaction after recognizing the MHC molecule. For example, at least one of the TCRs of the subject may recognize at least one molecule on the allogeneic T cell as being an "exogenous" MHC molecule of the present application. For example, after identifying as the exogenous source, the subject may produce the alloreaction due to the presence of the exogenous MHC molecule.

In the present application, the alloreaction may comprise activation of the subject's immune response. For example, the alloreactive response may comprise a TH-1 immune response. In the present application, recognition of the MHC molecule by at least one of the TCRs of the subject may promote a TH-1 immune response in the subject. The TH-1 immune response may promote the suppression of the proliferation of tumor cells in the subject, and/or slow down the course of the tumor.

In the present application, the alloreaction may comprise the risk of causing graft-versus-host disease (GVHD) in the subject by the allogeneic T cell. For example, the allogeneic T cell, as exogenous cells, may have the risk of causing graft-versus-host disease (GVHD) in the subject. However, the allogeneic T cell may be engineered to reduce this risk.

For example, the T cell product may have the ability to reduce the risk of the allogeneic T cell causing graft versus host disease (GVHD) in the subject.

In the present application, compared with the allogeneic T cell without engineering, the engineered allogeneic T cell may reduce the risk of graft versus host disease (GVHD) caused by the allogeneic T cell in the subject. For example, the engineering modification may reduce the risk of the allogeneic T cell causing the GVHD in the subject by destroying the structure and/or activity of the TCR in the allogeneic T cell.

In the present application, the engineering modification may comprise the following step: down-regulating the expression and/or activity of CD3-related genes, TRAC genes and/or TRBC genes in the allogeneic T cell.

In the present application, the CD3-related genes may comprise genes capable of encoding proteins selected from the group consisting of the gamma chain of CD3, the delta chain of CD3, the epsilon chain of CD3, the zeta chain of CD3 and the eta chainof CD3.

In the present application, compared with the wild-type allogeneic T cell, the expression and/or activity of CD3-related genes, TRAC genes and/or TRBC genes in the engineered allogeneic T cell may be downregulated.

In the present application, by engineering the expression and/or activity of the CD3-related genes, the formation of the CD3 complex may be hindered and/or the activity of the CD3 complex may be down-regulated.

In the present application, the engineering modification may comprise gene mutation and/or gene silencing. For example, the expression of CD3-related genes, TRAC genes and/or TRBC genes in the allogeneic T cell may be down-regulated through gene mutation and/or gene silencing (for example, through gene editing).

In the present application, the engineering may comprise administering to the allogeneic T cell one or more substances selected from the group consisting of: antisense RNA, siRNA, shRNA, CRISPR/Cas system, RNA editing system, RNA guide Endonucleases, zinc finger proteases, Mega-TAL nucleases, TALENs, Sleeping beauty transposon systems and Meganucleases.

In the present application, the engineering modification may use nuclease as long as the nuclease may achieve the engineering modification of the present application.

In the present application, the engineering modification may comprise administering a CRISPR/Cas system to the allogeneic T cell.

In the present application, the engineering modification may comprise administering to the allogeneic T cell a sgRNA targeting the exon portion of the CD3-related gene, the TRAC gene and/or the TRBC gene.

In the present application, at least one of the allogeneic T cell may be a universal T cell (U-T cell). For example, the allogeneic T cell may be adapted as universal T cells for administration to different subjects in need thereof. For example, the allogeneic T cell may be as universal T cells derived from any other source than the subject in need thereof. In some cases, the allogeneic T cell may be used as universal T cells and further prepared as universal CAR-T cells.

In the present application, at least one of the allogeneic T cell is modified to express an antibody or an antigen-binding fragment thereof, a chimeric antigen receptor (CAR), a cytokine and/or a chemokine.

In the present application, at least one of the allogeneic T cell may be modified to comprise nucleic acids encoding antibodies or antigen-binding fragments thereof, chimeric antigen receptors (CARs), cytokines and/or chemokines.

In the present application, the modification may comprise transfecting the nucleic acid into the allogeneic T cell.

In the present application, the transfection may comprise viral transfection and/or non-viral transfection. For example, viruses may be used for allogeneic T cell modification described herein. For example, the non-viral transfection may use plasmids.

In the present application, the plasmid may be a circular plasmid, a supercoiled plasmid or a linear plasmid. For example, the plasmid may be a circular plasmid or a supercoiled plasmid.

In the present application, the plasmid may be a DNA plasmid. For example, the DNA plasmid may be a double-stranded, closed-circle DNA molecule. For example, the DNA plasmid may be a double-stranded, linear DNA molecule. In the present application, the plasmid may be artificially synthesized.

In the present application, the plasmid may comprise a plasmid produced by bacteria. In the present application, the plasmid may comprise nanoplasmid and/or minicircle DNA vector.

In the present application, the plasmid may comprise ssDNA and/or dsDNA. In the present application, the ssDNA and/or dsDNA may be artificially synthesized.

In the present application, the plasmid may comprise nucleic acids encoding antibodies or antigen-binding fragments thereof, chimeric antigen receptors (CARs), cytokines and/or chemokines. The nucleic acid may comprise ssDNA and/or dsDNA. In the present application, the nucleic acid molecule may be artificially synthesized.

In the present application, the transfection may comprise stable transfection and/or transient transfection.

In the present application, the transient transfection may comprise electroporation. For example, the transient transfection may allow the nucleic acid to enter the allogeneic T cell for expression. In the present application, the transient transfection may comprise mRNA transfection. For example, the transient transfection may allow the nucleic acid (mRNA) to be electroporated into the allogeneic T cell for expression.

In the present application, the stable transfection comprises the use of transposon systems and/or the use of gene editing knock-ins.

In the present application, the gene editing method may be selected from one or more of the following groups: CRISPR/Cas system, RNA editing system ADAR, RNA-guided endonuclease, zinc finger protease, Mega-TAL nucleases, TALENs and Meganucleases.

In the present application, the gene editing method may be known to those skilled in the art, as long as the purpose of gene editing may be achieved, and is not limited to a specific method. In the present application, the gene editing method may be selected from one or more of the following groups: CRISPR/Cas system, RNA editing system ADAR, RNA-guided endonuclease, zinc finger protease, Mega-TAL nucleases, TALENs and Meganucleases. For example, the gene editing method may use the CRISPR/Cas system.

In the present application, the gene editing may comprise gene editing knock-out and/or gene editing knock-in. For example, the gene editing may comprise gene editing knock-in. For example, the CRISPR/Cas system may be used for the gene editing knock-in.

For example, the CRISPR-CAS system may comprise a type of clustered regularly interspaced short palindromic repeats (CRISPRs) and some functionally related proteins (CRISPR-associated, Cas). The Cas protein coding genes may comprise Cas9, Cas1, Cas2 and Csn2. For example, it may be Cas9. The CRISPR-CAS system (such as the CRISPR/Cas9 system) may have targeted cleavage specificity to DNA molecules.

For example, the gene editing knock-in may be a process of targeting the Cas protein to a specific DNA sequence in a cell to insert the DNA sequence. The gene editing knock-in may be a Cas protein (such as Cas 9 protein) mediated homologous recombination between the exogenous gene to be knocked-in in the donor plasmid and the specific DNA sequence in the targeted cell.

In the present application, the plasmid may be used as a donor plasmid in the gene editing knock-in. For example, the donor plasmid may be double-stranded DNA or single-stranded DNA. The donor plasmid may serve as a donor template for the HDR repair mechanism.

In the present application, the donor plasmid may comprise homology arms. For example, the homology arm may be homologous to the end of the exogenous gene or fragment thereof to be knocked in by gene editing. For example, the donor plasmid may comprise a 5' homology arm and/or a 3' homology arm. For example, the 5' end and/or 3' end of the nucleic acid molecule of the exogenous gene or fragment thereof may be respectively connected with the 5' end and/or 3' end of the position to be knocked in the group DNA of the cell to be transfected. The nucleic acid sequence has at least about 80%, at least about 85%, at least about 90%, at least about 91%, at least about 92%, at least about 93%, at least about 94%, at least about 95%, at least about 96%, at least about A nucleic acid sequence that is 97%, at least about 98%, at least about 99%, or 100% identical. For example, the homology arms may be 100-1000 bp in size. For example, the homology arm may be about 100 bp-1000 bp, may be about 150 bp-1000 bp, may be about 200 bp-1000 bp, may be about 300 bp-1000 bp, may be about 400 bp-1000 bp, may be about 800 bp-1000 bp.

In the present application, in the transfection mixture compriseing the plasmid, the content of the nucleic acid molecules or fragments thereof derived from the genome of the microorganism may account for about 10% (w/w) or less of the total content of the nucleic acid molecules of the transfection mixture (for example, may be about 10% (w/w) or less, about 9% (w/w) or less, about 8% (w/w) or less, about 7% (w/w) or less, about 6% (w/w) or less, about 5% (w/w) or less, about 4% (w/w) or less, about 3% (w/w) or less, about 2% (w/w) or less, about 1% (w/w) or less, about 9%o (w/w) or less, about 8%o (w/w) or less, about 7%o (w/w) or less, about 6%o (w/w) or less, about 5%o (w/w) or less, about 4%o (w/w) or less, about 3%o (w/w) or less, about 2%o (w/w) or less, about 1‰ (w/w) or less, about 0. 1‰ (w/w) or less, about 0.01%o (w/w) or less, about 0.001%o (w/w) or less).

In the present application, the size of the fragment of the nucleic acid molecule derived from the genome of the microorganism may be at least about 48 kb (for example, may be at least about 50 kb, at least about 100 kb, at least about 150 kb, at least about 200 kb, at least About 250 kb, at least about 300 kb, at least about 350 kb, at least about 400 kb, at least about 450 kb, at least about 500 kb, at least about 600 kb, at least about 700 kb, at least about 800 kb, at least about 900 kb, at least about 1 Mb, at least about 2 Mb, at least about 3 Mb, at least about 4 Mb, at least about 5 Mb, at least about 6 Mb, at least about 7 Mb, at least about 8 Mb, at least about 9 Mb, at least about 10 Mb, at least about 20 Mb, at least about 50 Mb, at least about 100 Mb, at least about 200 Mb or larger).

In the present application, in the transfection mixture comprising the plasmid, the content of the nucleic acid molecule or fragments thereof with a size of at least about 48kb (for example, may be at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) may account for less than about 10% (w/w) of the total nucleic acid molecule content of the transfection mixture (for example, may be about 10% (w/w) or less, about 9% (w/w) or less, about 8% (w/w) or less, about 7% (w/w) or less, about 6% (w/w) or less, About 5% (w/w) or less, about 4% (w/w) or less, about 3% (w/w) or less, about 2% (w/w) or less, about 1% (w/w) or less, About 9‰(w/w) or less, about 8‰(w/w) or less, about 7%o (w/w) or less, about 6%o (w/w) or less, about 5%o (w/w) or less, About 4%o (w/w) or less, about 3%o (w/w) or less, about 2%o (w/w) or less, about 1‰ (w/w) or less, about 0.1‰ (w/w) or less, about 0.01‰ (w/w) or less, about 0.001 %o (w/w) or less).

In the present application, the content of the nucleic acid molecule or fragment thereof with a size of at least about 48kb may be less than about 10%, less than about 9%, less than about 8%, less than about 7%, less than about 6%, less than about 5%, less than about 4%, less than about 3%, less than about 2%, less than about 1%, less than about 9‰, less than about 8‰, less than about 7‰, less than about 6‰, less than about 5‰, less than about 4‰, less than about 3‰, less than about 2‰, less than about 1‰, less than about 0.1‰, less than about 0.01‰, less than about 0.001‰, or even lower of the total content of nucleic acid molecules in the transfection mixture. For example, the content of the nucleic acid molecule or fragment thereof with a size of at least 48kb may be less than about 2% of the total content of nucleic acid molecules in the transfection mixture; it may be less than about 5‰ of the total content of nucleic acid molecules in the transfection mixture; it may be less than about 1‰ of the total content of nucleic acid molecules in the transfection mixture.

In the present application, the nucleic acid molecule or fragment thereof with a size of at least about 48kb (e.g., may be at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) and the plasmid are present in different nucleic acid molecules. For example, the nucleic acid molecule or fragment thereof with a size of at least about 48kb is free in the transfection mixture (the transfection mixture may comprise plasmid). For example, the nucleic acid molecule or fragments thereof with a size of at least about 48kb is free from the plasmid. The freedom may be present in a separated manner without attachment to the plasmid.

In the present application, the nucleic acid molecule or fragments thereof with a size of at least about 48kb may be derived from a microorganism. For example, the microorganism may be selected from one or more of the following group: bacteria, fungi, actinomycetes, mycoplasma, chlamydia, rickettsiae, and spirochetes.

In the present application, the microorganism may comprise Gram-negative bacteria. For example, the microorganism may be suitable for preparing a skeletal vector of the vector. For example, the microorganism may comprise *Escherichia coli.*

In the present application, the plasmid (e.g., linear plasmid) may be treated (e.g., by deoxyribonuclease, e.g., by exonuclease, e.g., Exonuclease V) to make the content of the nucleic acid molecule or fragments thereof with a size of at least about 48kb (e.g., may be a linear nucleic acid molecule or fragments thereof) and/or the content of the nucleic acid molecule or fragment thereof derived from the microbial genome (e.g., may be a linear nucleic acid molecule or fragments thereof) meet the requiremnet of the present application. In the present application, the treatment may not affect nucleic acid molecules with a circular structure.

In the present application, the plasmid may be treated with deoxyribonuclease (DNase). For example, after the treatment by the DNase, in the transfection mixture compriseing the plasmid, the content of nucleic acid molecules or fragments thereof derived from the genome of the microorganism may account for less than about 10% of the total content of nucleic acid molecules of the transfection mixture. For another example, after the treatment by the DNase, in the transfection mixture compriseing the plasmid, the content of the nucleic acid molecules or fragments thereof with a size of at least about 48kb may account for less than about 10% of the total content of nucleic acid molecules in the transfection mixture.

In the present application, the content of the nucleic acid molecules or fragments thereof in the transfection mixture may be reduced. In the present application, the reduction may refer to reducing the content of the nucleic acid molecules or fragments thereof in the transfection mixture to meet the requirements of the method of the present application.

In the present application, the reduction may comprise purifying the transfection mixture. That is, through the purification, the content of the nucleic acid molecules or fragments thereof in the transfection mixture may be reduced to meet the requirements of the method of the present application.

In the present application, the nucleic acid molecules or fragments thereof with a size of at least about 48kb may be removed through the purification; and/or the nucleic acid molecules or fragments thereof derived from the genome of microorganisms may be removed. For example, the purification may comprise reducing the content of the nucleic acid molecules or fragments thereof in the transfection mixture using methods for purifying DNA well known to those skilled in the art. In the present application, the purification may not affect the integrity and/or activity of the DNA of the plasmid. In the present application, the reduction may comprise purifying the transfection mixture using a reagent selected from the group consisting of: DNAase, SDS, TX-100, CTAB, and cesium chloride-ethidium bromide. For example, the reduction may use DNAase. For example, the use of DNAase may reduce the content of nucleic acid molecule or fragment thereof with a size of at least about 48 kb in the transfection mixture to meet the requirements of the method of the present application; and/or, reduce the content of nucleic acid molecule or fragment thereof derived from the microbial genome to meet the requirements of the method of the present application. For example, the DNAase may not affect the integrity and/or activity of the circular plasmid DNA.

In the present application, the reduction may comprise using a method selected from the following group: DNA synthesis of the plasmid according to the present application and HPLC detection of the transfection mixture according to the present application. For example, direct DNA synthesis can be performed based on the nucleotide sequence of the plasmid, which can result in a plasmid containing only the nucleotide sequence of the plasmid. For example, the transfection mixture of the present application can be detected by HPLC, and based on the HPLC detection results, the characteristic peak corresponding to the plasmid can be selected, and the component corresponding to the characteristic peak can be collected, which can result in a plasmid containing only the nucleotide sequence of the plasmid.

In the present application, the antibody or antigen-binding fragment thereof may comprise bispecific antibody.

In the present application, the antibody or antigen-binding fragment thereof may comprise bispecific T cell engager (BiTE).

In the present application, the CAR may comprise an antigen-binding domain, a hinge region, a transmembrane domain, a costimulatory domain and a signaling domain. For example, the CAR may be composed of an antigen-binding domain, a hinge region, a transmembrane domain, a costimulatory domain, and a signaling domain.

In the present application, the antigen-binding domain of the CAR can specifically bind to one or more tumor-associated antigens.

In the present application, the antigen-binding domain of the CAR can specifically bind GPC3 and CD73.

In the present application, the antigen-binding domain of the CAR can specifically bind GPC3 and CD147.

In the present application, the antigen-binding domain can specifically bind to a tumor-associated antigen (TAA).

In the present application, the antigen-binding domain may be selected from a group consisting of: monoclonal antibodies, polyclonal antibodies, human antibodies, humanized antibodies, single domain antibodies, Nanobodies and antigen-binding fragments thereof.

In the present application, the hinge region may comprise a polypeptide from a protein selected from the following group consisting of CD28, HLA-A, HLA-B, HLA-C, HLA-G, HLA-DQ, HLA-DP, HLA-DR, CD8, Fc, IgG, IgD, 4-1BB, CD4, CD27, CD7 and PD1.

In the present application, the transmembrane domain may comprise a polypeptide from a protein selected from the following group consisting of: CD27, CD7, PD1, TRAC, TRBC, CD28, CD3e, CD45, CD4, CD5, CD8, CD9, CD16, CD22, CD33, CD37, CD64, CD80, CD86, CD134, CD137 and CD154.

In the present application, the costimulatory domain may comprise a polypeptide from a protein selected from the following group consisting of: CD28, CD137, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS and ligand of CD83.

In the present application, the signaling domain may comprise a polypeptide from a protein selected from the following group consisting of: CD3ζ, FcRy (FCER1G), FcyRIIa, FcRβ (FcεR1b), CD3γ, CD3δ, CD3ε, CD79a, CD79b, DAP10, DAP12, Igα receptor, Igβ receptor, BLV gp30 (bovine leukemia virus gp30), EBV (Ep-stein-Barr virus) LMP2A, Simian Immunodeficiency Virus PBj14 Nef and immune receptor tyrosine activation motif (ITAM).

In the present application, the cytokine may be selected from the following group consisting of: IL-1, IL-7, IL-12, IL-13, IL-15, IL-16, IL-17A, IL-17F, IL-18, IL-21, IL-23, TNFα, CXCL1, CD38, CD40, CD69, IgG, IP-10, L-17A, MCP-1 and PGE2. In some cases, the allogeneic T cell can produce cytokines (such as IL-10, IL-2, IL-4, IL-5, IL-6, IL-7, IL-8 and/or IL-10) after co-incubation with target cells, such as tumor cells. For example, the modified allogeneic T cell may express cytokines other than those described above that may be expressed by themselves.

In the present application, the chemokine may be selected from the following group consisting of: CCL1-CCL28 and CXCL1-CXCL17.

In the present application, at least one of the allogeneic T cell may express and/or comprise a chimeric antigen receptor (CAR) after transfection. In the present application, at least one of the allogeneic T cell may express and/or comprise a bispecific T cell adapter (BiTE) after transfection.

In the present application, at least one of the allogeneic T cell may comprise at least one (e.g., at least 1, at least 2, at least 3 or more) plasmids comprising the nucleic acid after transfection.

In the present application, at least one of the allogeneic T cell may express more than two proteins.

In the present application, at least one of the allogeneic T cell may express and/or comprise a chimeric antigen receptor and one or more cytokines after transfection. For example, at least one of the allogeneic T cell may be transfected to express and/or comprise a chimeric antigen receptor, as well as IL15 and IL-21.

For example, the nucleic acid molecule encoding the CAR may comprise the nucleic acid sequence shown in SEQ ID NO. 2.

For example, the nucleic acid molecule encoding the CAR may comprise the nucleic acid sequence shown in SEQ ID NOs. 11 or 12.

In the present application, the content of the at least one allogeneic T cell in the T cell product may be about 0.5%, about 1%, about 2%, about 3%, about 4%, about 5%, About 6%, about 7%, about 8%, about 9%, about 10%, about 11%, about 12%, about 15%, about 20%, about 25%, about 30%, about 35%, about 40%, about 45%, about 50%, about 55%, about 60%, about 65%, about 70%, about 75%, about 80%, about 85%, about 90%, about 95% or about 100%.

In the present application, at least two of the allogeneic T cells expressing different chimeric antigen receptors (CARs) and/or cytokines may be mixed proportionally.

In some cases, the T cell product may be a mixture of the allogeneic T cells, depending on the purpose of treatment for the subject in need thereof. For example, in one aspect, the allogeneic T cell may be derived from at least one of the donors.

For example, in another aspect, at least one of the allogeneic T cell may be modified to express at least one protein required by the subject. In some cases, at least one of the allogeneic T cell may be modified to express a protein, for example, may express a chimeric antigen receptor. In some cases, at least one of the allogeneic T cell may be modified so that the same allogeneic T cell expresses at least 2 (e.g., at least 2, at least 3, at least 4 or more) proteins. For example, one of the allogeneic T cell may express a chimeric antigen receptor and simultaneously express a cytokine. As another example, one of the allogeneic T cell may express a chimeric antigen receptor and simultaneously express a cytokine and a bispecific antibody. In some cases, the T cell product may comprise the allogeneic T cells expressing different types and amounts of proteins. For example, according to the purpose of the subject, the allogeneic T cell derived from different donors and capable of expressing different types and different amounts of proteins may be mixed in the T cell product in certain proportions. In some cases, the allogeneic T cell of different types and sources may be "customized" according to the purpose of the subject and mixed in the desired proportion.

In some cases, the allogeneic T cell is engineered as described herein to have a reduced ability to cause graft versus host disease (GVHD) in the subject. On this basis, at least one of the allogeneic T cell may be further modified to express at least one protein, for example, may express a chimeric antigen receptor. On the basis that the allogeneic T cell are universal T cells, in some cases, at least one of the allogeneic T cell may be modified so that the same allogeneic T cell expresses at least 2 (for example, at least 2, at least 3, at least 4 or more) proteins. For example, a universal type of the allogeneic T cell may express a chimeric antigen receptor and simultaneously express a cytokine. As another example, a universal type of allogeneic T cell can express a chimeric antigen receptor and simultaneously express a cytokine and a bispecific antibody. In some cases, the T cell product may comprise these universal allogeneic T cells expressing different types and amounts of proteins. For example, according to the purpose of the subject, in the T cell product, these universal allogeneic T cell derived from different donors and capable of expressing different types and different amounts of proteins may be mixed in a certain proportion. In some cases, different types of universal allogeneic T cell from different sources may be "customized" according to the purpose of the subject and mixed in the desired proportion.

In some cases, the allogeneic T cell derived from different donors (such as the engineered allogeneic T cell of the present application) may be mixed first, and these mixed cells may be respectively transfected (for example, electroporated, for example, electroporated mRNA) with at least one nucleic acid encoding a protein. For example, these mixed cells may be transfected with nucleic acid molecules encoding chimeric antigen receptors and/or nucleic acids encoding cytokines. These transfected cells can then be mixed in the desired proportions depending on the subject's purpose.

In some cases, the allogeneic T cell (such as the engineered allogeneic T cell of the present application) may be respectively transfected (such as electroporation, such as electroporation of mRNA) with at least one nucleic acid molecule encoding a protein. For example, the allogeneic T cell may be transfected with nucleic acid molecules encoding chimeric antigen receptors, and/or nucleic acid molecules encoding cytokines. These transfected cells (e.g., may be derived from at least 2 donors) can then be mixed in the desired proportions depending on the subject's purpose.

In the present application, the T cell product may comprise T cells derived from the subject (i.e., "autologous" T cells). In some cases, the T cells derived from the subject may be modified to express at least one protein required by the subject (e.g., may express the chimeric antigen receptor).

Compared with allogeneic T cell derived from the subject, the T cell product of the present application may cause a more vigorous immune response in the subject.

In the present application, the level of protein expressed by the modified allogeneic T cell of the T cell product may not be affected by the allogeneic source of the allogeneic T cell.

In the present application, the T cell product may also comprise other types of T cells. In the present application, the T cell product may also comprise T cells derived from the subject's autologous source. In the present application, the T cell product may further comprise one or more other T cells. For example, the other T cells may be modified T cells. For example, the other modified T cells have a reduced ability to cause an alloreaction in the body compared with corresponding unmodified T cells. For example, the other modified T cells have reduced expression and/or activity of MHC molecules compared with corresponding unmodified T cells. For example, the other T cells may be allogeneic.

For example, the modified allogeneic T cell may comprise T cells with mutated MHC-I. For example, the modified T cells may comprise MHC-I knockout T cells. For example, the modified T cells may comprise T cells that have MHC-I knocked out but express HLA-E or HLA-G. For example, the modified T cells may comprise any UCART known in the art. For example, the modified T cells may comprise T cells with CD52 gene knocked out.

### Pharmaceutical Compositions and Use

In another aspect, the present application provides a pharmaceutical composition comprising the T cell product of the present application and a pharmaceutically acceptable excipient.

In the present application, the excipients may comprise carriers. For example, the carrier may comprise phosphate-buffered saline solution, water, emulsions (such as oil/water emulsions), various moisturizers, sterile solutions, and liposomes.

In the present application, the excipients may be suitable for injection.

In the present application, the excipients may be suitable for intratumoral injection and/or systemic injection. For example, the excipients may comprise sterile aqueous or non-aqueous solutions, suspensions, and emulsions. Non-aqueous solvents may be, for example, propylene glycol, polyethylene glycol, vegetable oils (e.g., olive oil), and injectable organic esters (e.g., ethyl oleate).

In the present application, the excipients may comprise preservatives and other additives, such as antimicrobials, antioxidants, chelating agents and/or inert gases, etc.

In the present application, the dosage of the pharmaceutical composition may be determined by the participating physician and clinical factors. As is well known in the art of medicine, the dosage for any given patient depends on many factors, including the patient's size, body surface area, age, the specific compound being administered, gender, time and route of administration, general medical condition, and other medications administered simultaneously.

In some cases, the pharmaceutical compositions may comprise other pharmaceutically active ingredients. The type and/or content of the pharmaceutical active ingredients may be determined according to the condition of the subject.

On the other hand, the present application provides a kit comprising the T cell product of the present application and an injection equipment.

For example, the injection equipment may comprise a syringe. In the intratumoral injection, the needle of the syringe may be inserted into the subcutaneous area outside the tumor first, and then into the tumor. The intratumoral injection may comprise multiple point injections.

In another aspect, the present application provides the use of the T cell product of the present application, the pharmaceutical composition of the present application and/or the kit of the present application in the preparation of a medicament for prevention, alleviation, and treatment of tumor.

In the present application, the tumor comprises solid tumors. For example, the tumor may comprise lung cancer.

In the present application, the medicament is formulated for injection.

In the present application, the drug is formulated for intratumoral injection.

In another aspect, the present application provides a method for improving the tumor microenvironment, comprising the following step: administering to a subject the T cell product of the present application, the pharmaceutical composition of the present application, and/or the kit of the present application.

In another aspect, the present application provides a method for preventing, alleviating, and treating tumors, comprising the following step: administering to a subject the T cell product of the present application, the pharmaceutical composition of the present application, and/or the kit of the present application.

In the present application, the administration comprises injection.

In the present application, the administration comprises intratumoral injection.

In the present application, the administration comprises more than one intratumoral injection. The number of intratumoral injections may be determined according to the condition of the subject.

In another aspect, the present application provides a T cell product of the present application, a pharmaceutical composition of the present application and/or a kit of the present application for use in prevention, alleviation and treatment of tumor.

Without intending to be limited by any theory, the following examples are only for illustrating various technical solutions of the invention of the present application, and are not intended to limit the scope of the invention of the present application.

### Examples

### Example 1 Engineered and modified allogeneic T cell can effectively knock out CD3-positive cells

### 1.1 Engineered allogeneic T cell

(1) Dynal Beads and CD3 positive cells were activated in the X-vivo15+20U/ml culture medium at a ratio of 1:1. T cells derived from different donors (Donor 1-Donor 3) were collected and name them respectively as Donor 1-T cells, Donor 2-T cells and Donor 3-T cells; the steps of T cell collection are as follows:
(2) After 2-3 days of activation, Cas9/CRISPR/sgRNA system was used to knock out CD3 in donor 1-T cells, donor 2-T cells and donor 3-T cells respectively (the nucleotide sequence of sgRNA targeting human CD3-related genes is shown as SEQ ID NO. 1, and each of the 5' end and 3' end has 3 thio and oxymethyl modifications, purchased from GenScript Biotechnology, Nanjing);
(3) Two days after CD3 knockout, CD3⁺ immunomagnetic beads were used (EasySep ^{™} Human CD3 Positive Selection Kit II ((Catalog #17851, purchased from Stemcell Technologies) to further screen the cells knocked out in step (2) for removing CD3-positive cells.

After the treatment in steps (2) and (3), cell flow cytometry was used to detect the proportion of CD3-positive cells. The results are shown in Table 1. The cells were cultured for another 5-7 days for further genetic modification.

**Table 1**

| | After treated in step (3) |
|---|---|
| Donor 1-T cells | 0.2 |
| Donor 2-T cells | 0.3 |
| Donor 3-T cells | 0.3 |

1.2
The preparation of mRNA was achieved using the Hiscribe T7 ARCA mRNA kit (NEB, #E20605) and the preparation procedures accompanied by the product. The plasmids were coined into pcDNA3.1. Linearization of plasmids was achieved via XhoI.

The nucleic acid molecules (mRNAs) comprising those encoding CAR were transfected with donor 1-T cells, donor 2-T cells, and donor 3-T cells prepared by example 1.1 that had been knocked out and removed of CD3-positive cells, respectively, by electroporation.

Wherein, the nucleotide sequence of the nucleic acid molecule encoding CAR targeting GPC3is shown in SEQ ID NO. 2. These modified cells are named D1-T, D2-T and D3-T respectively.

1.3
The backbone plasmid of the GPC 3 CAR-2A-IL15-2A-IL21 plasmid is pUC57-mini-Amp or pUC57-mini-Kan, cloned into nucleic acid molecules encoding GPC3 CAR-2AIL15-2A-IL21 (wherein the nucleotide sequence encoding GPC3 CAR-2A-IL15-2A-IL21 is shown in SEQ ID NO. 11). Meanwhile, these plasmids comprise nucleic acid sequences homologous to part of the human TRAC gene. Chemically synthesized sgRNA was designed to target human TRAC gene (5', the nucleotide sequence is as shown in SEQ ID NO. 1, and each of the 5' end and 3' end has 3 thio and oxymethyl modifications, purchased from Genscript Biotechnology, Nanjing). The Cas9 protein was purchased from Yiqiao China (Product No.: 40572-A08B).

CRISPR/sgRNA system was used to electroporate the prepared plasmid into human T cells that had been activated with Dynal bead for 2-3 days to obtain CART cells of 3 donors D1, D2, and D3 that were knocked in with GPC-3 CAR-2A-IL15-2A-IL21 (D5-CART, D6-CART, D7-CART, in which the CD3 positive rate is as shown in Table 2 after knocking in, and cryopreserved. After recovery, CD3⁺ immunomagnetic beads (EasySep^{™} Human CD3 Positive Selection Kit II ((Catalog #17851, purchased from Stemcell Technologies)) were used to further screen the knock-in CAR cells to remove CD3-positive cells, and obtain the remaining knock-in/CD3 removed CD3-positive cells, as shown in table 2.

**Table 2 three donors with knock-in CAR, remaining CD3⁺ cells after removing CD3⁺ T cells.**

| | CD3⁺ | |
|---|---|---|
| | After knocking in | GPC 3 CAR-2A-IL15-2A-IL21 Knock-in /CD3 removed |
| D5-CART | 12% | 0.3% |
| D6-CART | 5% | 0.1% |
| D7-CART | 10% | 0.1% |

### Example 2 Allogeneic T cell cause alloreaction

Alloreaction in allogeneic T cells was investigated by using Calcein-AM-labeled, unirradiated PBMCs derived from 4 different donors.

2.1
The D1-T, D2-T and D3-T prepared in Example 1.2 were irradiated (irradiation conditions: 60 Gy X-rays, RS2000 Pro, Rad Source, United States), and were co-incubated with congeneric PBMCs (i.e., D1-PBMC, D2-PBMC, D3-PBMC) from their respective sources or allogeneic D4-PBMCs, respectively;

In addition, D1-T, D2-T and D3-T were mixed, and the mixed cells were co-incubated with D4-PBMC to ensure maximum alloreaction.

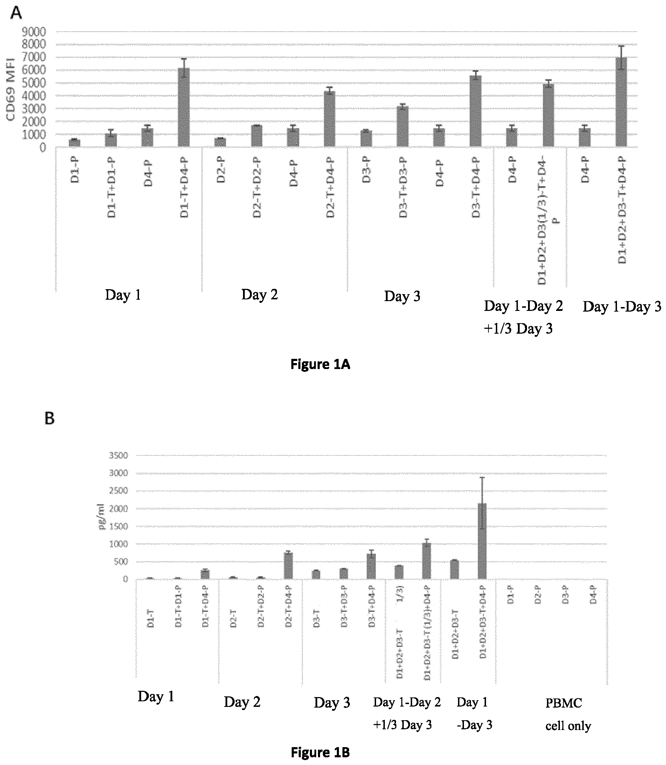

After overnight co-incubation, flow cytometry was used to detect the MFI value of T cell CD69 in PBMC. The results are shown in Figure 1A (PBMC cells are represented by P in the figure), wherein D1-P represents PBMC cells derived from donor 1. D1-T is the CART cells derived from donor 1 expressing GPC3 CAR prepared in Example 1. D1-P+D1-T represents the result obtained by mixing the same amounts of D1-T and D1-P, and the total amount of D1-P+D1-T is the same as the D1-P on the left. Similarly, D1+D2+ D3 (1/3)-T represents the result of mixing the same amounts of D1-T, D2-T and D3-T, each of which accounts for 1/3, and the total amount of D1+D2 +D3 (1/3)-T and D4-P is the same as the amount of D4-P on the left. Similarly, D1+D2+ D3-T represents the result obtained by mixing D1-T, D2-T and D3-T, and the total amount ofD1+D2+ D3-T and D4-P is the same as the amount of D4-P on the left. The other abscissas in Figure 1A have similar meanings.

The results in Figure 1A illustrate that the MFI of CD69 produced by the interaction between allogeneic cells (such as D1-T+D4-PBMC) is significantly higher than that of CD69 produced by the interaction between homologous cells (such as D1-T+D1-PBMC). The degree of T/NK cell activation generated from a mixture of CART cells derived from these three different donors mixed with PBMC from a 4th donor (i.e., allogeneic to these three donors) can illustrate that the multi allogeneic donor-derived CART can ensure a greater degree of alloreactivity as described herein.

2.2
The D1-T, D2-T and D3-T prepared in Example 1.2 were irradiated (irradiation conditions: 60 Gy X -rays, RS2000 Pro, Rad Source, United States), and were c co-incubated with PBMC (i.e. D1-PBMC, D2-PBMC, D3-PBMC) from their respective sources or allogeneic D4-PBMC;

Additionally, D1-T, D2-T, and D3-T were mixed, and the mixed cells were co-incubated with D4-PBMC.

After overnight co-incubation, CBA was used to detect the IFN-γ content in the cell culture supernatant. The testing steps follow the procedures accompanied by the product. The results are shown in Figure 1B (PBMC cells are represented by P in the figure). The meaning of the abscissa in Figure 1B is the same as that in Figure 1A.

The results in Figure 1B illustrate that the content of IFN-γ produced by the interaction between allogeneic cells (such as D1-T+D4-PBMC) is significantly higher than that produced by the interaction between homologous cells (such as D1-T+D1-PBMC). Due to the significant upregulation of IFN-γ expression produced by the mixing of CART cells from three different donor sources with PBMCs from the 4th donor (i.e., allogeneic to these three donors), it can be seen that CART from multiple allogeneic donors can ensure a greater degree of allogeneic response as described in this application.

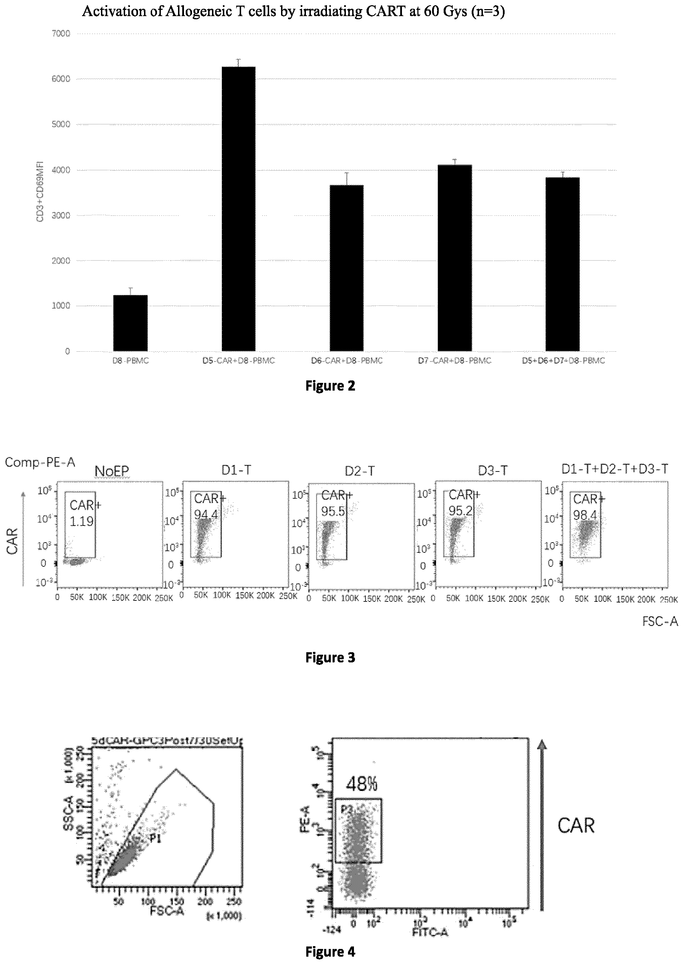

2.3
D5-CART, D6-CART and D7-CART prepared in Example 1.3 were irradiated (irradiation conditions: 60 Gy X-rays, RS2000 Pro, Rad Source, United States), and were individually or separately mixed to incubate with allogeneic D8-PBMC overnight to generate an alloreaction. Flow cytometric was used to measure MFI of CD69 of T cell in D8-PBMC. The results are shown in Figure 2. The results show that when only D8-PBMC is used, the average fluorescence value of CD69 of T cells in D8-PBMC is the lowest. The average fluorescence intensity of CD69 of T cells in D8-PBMC was significantly enhanced, when incubated overnight with irradiated D5-CART, D6-CART, D7-CART, or D5-CART +D6-CART+D7-CART, indicating that the alloreaction activated T cells.

### Example 3 Mixture of T cells derived from different donors does not affect the ability to express molecules by electroporation

3.1
Flow cytometry was used to detect the expression of CAR in the D1-T, D2-T and D3-T prepared in Example 1.2, as well as the mixed cells of D1-T, D2-T and D3-T.

The results are shown in Figure 3. Wherein, NoEP serves as the control group, which represents T cells that are not electroporated and do not comprise GPC3 CAR.

The results in Figure 3 illustrate that there was no significant difference in the amount of CAR expressed in D1-T, D2-T, D3-T and mixed cells of D1-T, D2-T and D3-T.

### 3.2 Use of non-viral localization knock-in method to achieve long-term expression of CAR molecules

The backbone plasmid of GPC-3 CAR plasmid is pUC 57-mini-Amp or pUC 57-mini-Kan, cloned into uucleic acid molecules encoding a CAR targeting GPC-3. Meanwhile, these plasmids comprise nucleic acid sequences homologous to part of the human TRAC gene. Design of chemically synthesized sgRNA targeting the human TRAC gene (5', whose nucleotide sequence is shown in SEQ ID NO. 1, and each of the 5' end and 3' end has 3 thio and oxymethyl modifications, purchased from GenScript Biotechnology, Nanjing). The Cas9 protein was purchased from Yiqiao China (Product No.: 40572-A08B). This plasmid is named anti-GPC-3 CAR plasmid, which comprises nucleic acid sequence as shown in SEQ ID NO. 2.

The prepared GPC-3 CAR plasmid was electroporated into human T cells that had been activated with Dynal bead for 2-3 days to obtain GPC-3 CART cells that were knocked in with anti-GPC-3 CAR.

The GPC-3 CART cells may be activated by Huh-7 target cells and survive long-term in *vitro* for more than 60 days. The expression level of CAR in CART after 60 days of *in vitro* amplification is shown in Figure 4.

The results in Figure 4 illustrate that GPC-3 CART cells can still have high expression level after being cultured *in vitro* for 60 days.

### 3.3 T cells can express CAR and other molecules simultaneously

Both nucleic acid molecules (mRNA) encoding CAR and nucleic acid molecules (mRNA) encoding cytokines or chemokines were transfected into T cells that were activated for 5-9 days using Dynal Bead cells at a 1:1 ratio using electroporation.

Wherein, the nucleotide sequence of the nucleic acid molecule encoding CAR targeting GPC 3is shown in SEQ ID NO. 2.

The nucleotide sequence of the nucleic acid molecule encoding the cytokine IL12a is shown in SEQ ID NO. 8.

The nucleotide sequence of the nucleic acid molecule encoding the cytokine IL12b is shown in SEQ ID NO. 9.

The nucleotide sequence of the nucleic acid molecule encoding the cytokine IL1b is shown in SEQ ID NO. 5.

The nucleotide sequence of the nucleic acid molecule encoding the chemokine CCL5 is shown in SEQ ID NO. 6.

The nucleotide sequence of the nucleic acid molecule encoding the chemokine CXCL10 is shown in SEQ ID NO. 7.

Wherein, those that simultaneously express GPC3 CAR and IL12a, IL12b, IL1b, CCL5 and CXCL10 are named GPC3+cytokine cells.

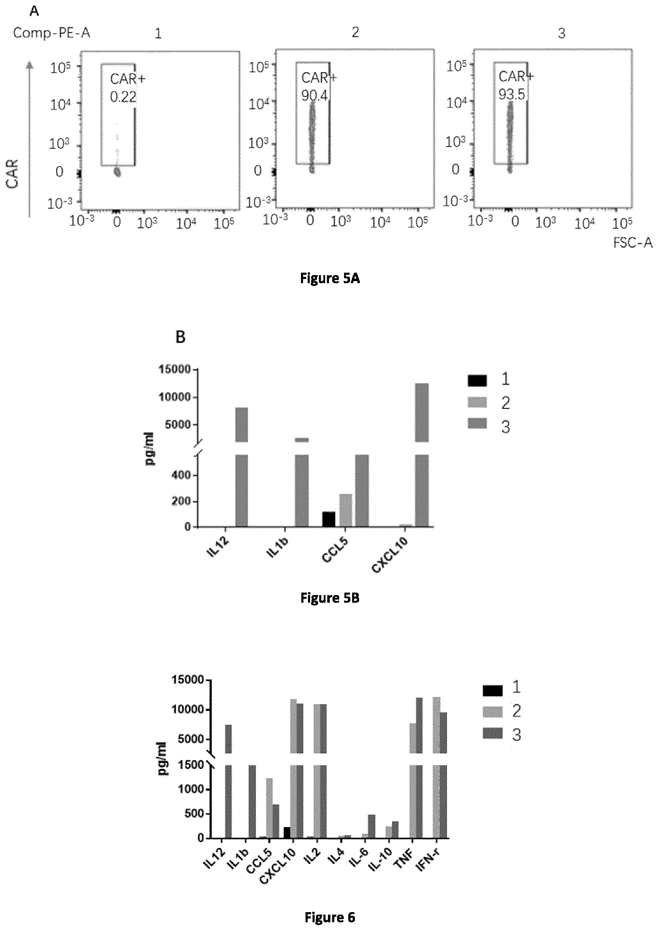

Flow cytometry was used to detect the expression of CAR in each cell; CBA was used to detect the expression of cytokines or chemokines in the culture supernatant of each cell; the results are shown in Figure 5A and Figure 5B respectively.

Figure 5A, 1-3 respectively show expression levels of CAR in: T cells that have not been electroporated and do not comprise GPC3 CAR; T cells prepared in Example 1 that are only electroporated with GPC3 CAR; GPC3+cytokine cells prepared in Example 3.

Figure 5B, 1-3 respectively represent expression levels of cytokines in cell culture supernatants of: T cells that have not been electroporated and do not comprise GPC3 CAR; T cells prepared in Example 1 that are only electroporated with GPC3 CAR; and GPC3+cytokine cells prepared in Example 3.

3.4
T cells that are not electroporated and do not comprise GPC3 CAR; T cells prepared in Example 1.2 that are only electroporated with GPC3 CAR; the GPC3+ cytokine cells prepared in Example 3 were respectively mixed and incubated with HepG2 target cells at an effect-target ratio of 2:1 to. After overnight, CBA was used to detect the expression of cytokines or chemokines in the culture supernatant of each cell; the results are shown in Figure 5. In Figure 6, 1-3 respectively represent: 1: T cells that have not been electroporated and do not comprise GPC 3 CAR; 2: T cells that are only electroporated with GPC3 CAR prepared in Example 1.2; 3: GPC3+cytokine cells prepared in Example 3.

### Example 4 Detection of specific killing function against tumor cells

4.1
The nucleic acid molecule (mRNA) comprising those encoding CAR were transfected using electroporation into T cells activated for 5-9 days at a ratio of Dynal Bead: cells =1:1, wherein CAR targets GPC3 and the nucleotide sequence of the nucleic acid molecule encoding the CAR is shown in SEQ ID NO. 2. The electroporated cells were mixed with target cells SK-HEP-1, Huh7 or HepG2 at a certain effect-target ratio. Their killing efficiencies were measured respectively and flow cytometry was used to detect the expression of GPC3 on the target cell surface.

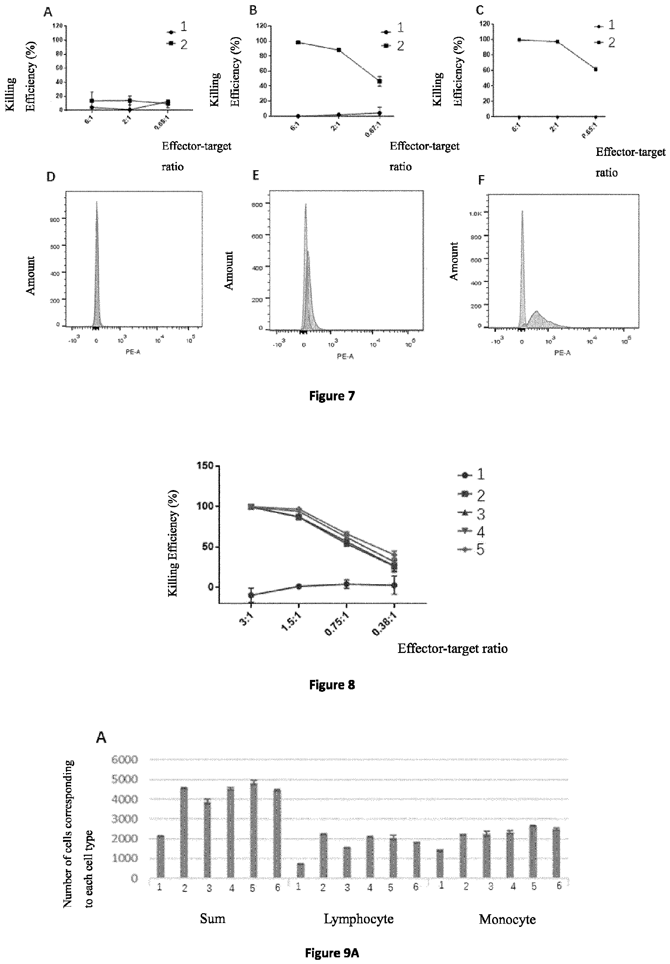

The results are shown in Figures 7A-7F respectively, wherein Figures 7A-7C respectively show the killing efficiency of target cells SK-HEP-1, Huh7 or HepG2; Figures 7D-7F respectively show the expression of GPC3 on the surface of the target cells SK-HEP-1, Huh7 and HepG2. Wherein, 1-2 respectively represent: 1: T cells that have not been electroporated and do not comprise GPC3 CAR; 2: T cells that were electroporated with CAR-mRNA.

The results in Figure 7 illustrate that the CART cells obtained by electroporation can express CAR and can specifically kill tumor cells.

4.2 CART cells from different donor sources or their mixtures thereof have similar functions of target cell killing

The D1-T, D2-T and D3-T prepared in Example 1.2, and the mixed cells of D1-T, D2-T and D3-T were respectively mixed with the target cell HepG2 at a certain effect-target ratio. Wherein, the target cell HepG2 was labeled with Calcein-AM.

Their killing efficiency was measured by flow cytometry.

The results are shown in Figure 8. In Figure 8, 1-5 respectively represent 1: T cells that have not been electroporated and do not comprise GPC3 CAR; 2: D1-T; 3: D2-T; 4: D3-T; 5: mixed cells of D1-T, D2-T and D3-T.

The results in Figure 8 illustrate that electroporation of allogeneic T cell derived from a single donor and electroporation of allogeneic T cell derived from a mixture of multiple donors can achieve the same function of target cell killing.

### Example 5 Detection of the ability to activate immune response after killing tumor cells

### 5.1 Recruit immune cells

The D1-T, D2-T and D3-T prepared in Example 1.2, as well as the mixed cells of D1-T, D2-T and D3-T were respectively used. Control cells: T cells that are not electroporated and did not comprise GPC3 CAR; and positive control cells: T cells electroporated with an excess of GPC3 CAR, respectively, co-incubated with the target cell HepG2 at an effect-target ratio of 6:1.

The supernatant was collected the next day, and 500 µL of supernatant from each group was added to the lower chamber of the Tranwell. Fresh PBMC were labeled with Calcein - AM, and the concentration was adjusted to 1e7/mL. 100 µL was added with the upper chamber of Tranwell and placed in the incubator for 1.5 hours, and then the number of cells subjected to chemotaxis in the lower chamber was detected, and the percentage of each type was detected.

The results are shown in Figures 9A-9B respectively, wherein 1-6 respectively represent the result of: control cells, D1-T, D2-T, D3-T, mixed cells of D1-T, D2-T and D3-T, and positive control cells..

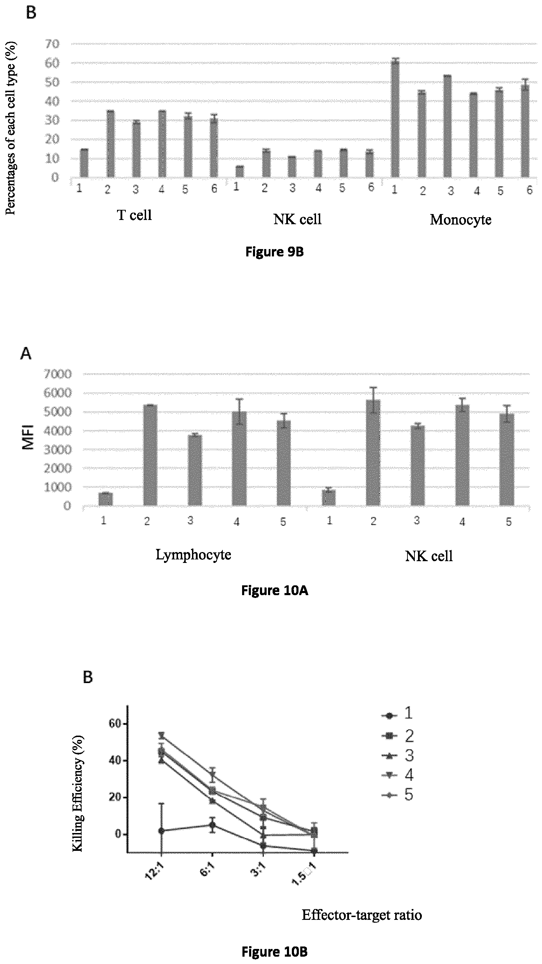

The results in Figure 9 show that the supernatants obtained after killing tumor cells from CART cells electroporated from a single donor source after co-incubating with target cells and CART cells electroporated from a mixture of multiple donors after co-incubating with target cells (the supernatant may comprise a variety of cytokines) both can recruit immune cells, and their ability to recruit immune cells are similar.

### 5.2 Ability to activate immune cells

The steps are the same as in Example 5.1.

The supernatant was collected the next day. 2e5 Calcein- AM-labeled PBMC cells were added with 100 µL of the supernatant of each group, and after culturing overnight, mean fluorescence intensity (MFI) of CD69 T cells and NK cells in PBMC was measured by flow cytometry.

The results are shown in Figure 10A. Wherein, 1-5 respectively represent: control cells, D1-T, D2-T, D3-T, mixed cells of D1-T, D2-T and D3-T. The control cells are T cells that have not been electroporated and do not comprise GPC3 CAR.

The results in Figure 10A show that the supernatants obtained after killing tumor cells from CART cells electroporated from a single donor source after co-incubating with target cells and CART cells electroporated from a mixture of multiple donors after co-incubating with target cells (the supernatant may comprise a variety of cytokines) both have ability to activate immune cells with comparative level.

### 5.3 Tumor cells killing

The D1-T, D2-T and D3-T prepared in Example 1.2, as well as the mixed cells of D1-T, D2-T and D3-T, and the control cells were co-incubated with the target cell HepG2 at an effective-to-target ratio of 6:1 respectively. The supernatant was collected after overnight. The control cells are T cells that have not been electroporated and do not comprise GPC3 CAR.

1.2e6 PBMC cells were repectively added with 600 µL of the supernatant collected in each group. After overnight culture, PBMCs were collected and used to perform K562 killing assay to verify whether PBMC were activated in the collected supernatant.

The results are shown in Figure 10B, wherein 1-5 respectively represent: control cells, D1-T, D2-T, D3-T, mixed cells of D1-T, D2-T and D3-T.

The results in Figure 10B show that PBMCs cultured by the supernatants obtained after killing tumor cells from CART cells electroporated from a single donor source after co-incubating with target cells and CART cells electroporated from a mixture of multiple donors after co-incubating with target cells (the supernatant may comprise a variety of cytokines) have similar killing abilities to K562, proving that these supernatants have the ability to activate PBMC.

### Example 6 Preparation and Characterization of "Armed" Allogeneic T cell

### 6.1 Preparation

The mixture of CD3-knockout donor 1-T cells, donor 2-T cells, and donor 3-T cells prepared in Example 1.1 were all electrotransfected using nucleic acid molecules (mRNA) encoding CAR and encoding cytokines.

Wherein, CAR targets GPC3, and the nucleotide sequence of the nucleic acid molecule encoding CAR is shown in SEQ ID NO. 2.

The nucleotide sequence of the nucleic acid molecule encoding the cytokine IL12a is shown in SEQ ID NO. 8.

The nucleotide sequence of the nucleic acid molecule encoding the cytokine IL12b is shown in SEQ ID NO. 9.

The nucleotide sequence of the nucleic acid molecule encoding the cytokine IL1b is shown in SEQ ID NO. 5.

The nucleotide sequence of the nucleic acid molecule encoding BiTE is shown in SEQ ID NO. 3.

Cells that express GPC3 CAR and IL12, IL1b, and BiTE simultaneously after transfection are named GPC3+IL12+IL1b+BiTE-1-T cells, or "Armed"-T cells.

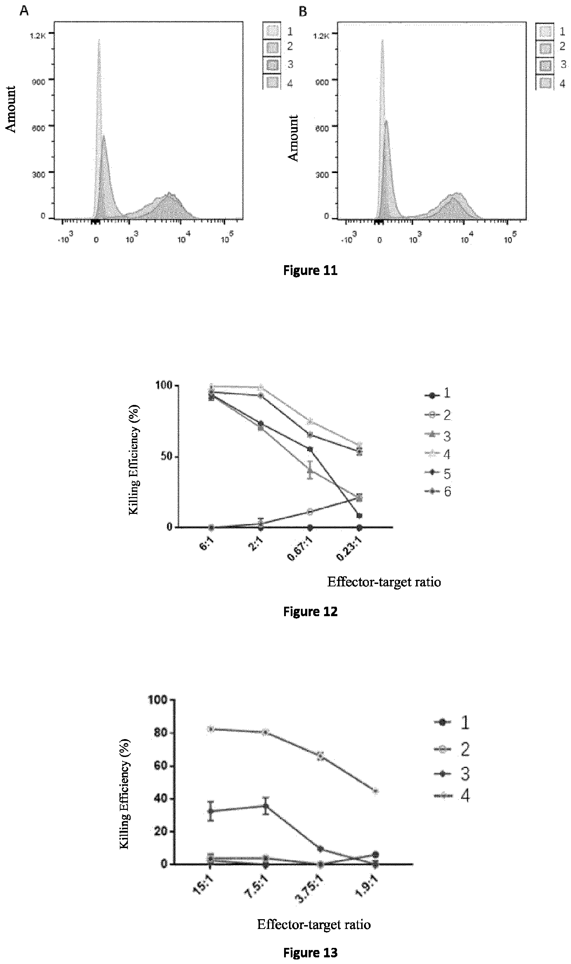

Flow cytometry was used to detect the expression of CAR in the mixed cells of D1-T, D2-T and D3-T cells prepared in Example 1.2 and the "Armed"-T cells prepared in Example 6.1; the results were respectively shown in Figure 11A and Figure 11B. Wherein, in Figure 11A, 1-4 respectively represent the expression levels of CAR of: control cells, after electroporation of mixed cells of D1-T, D2-T and D3-T cells for 2 hours, electroporation of mixed cells of D1-T, D2-T and D3-T cells for 1 day, electroporation of mixed cells of D1-T, D2-T and D3-T cells for 3 days; in Figure 11B, 1-4 represent the expression levels of CAR of: control cells, after electroporation of "Armed"-T cells prepared in Example 6.1 for 2 hours o, electroporation of "Armed"-T cells prepared in Example 6.1 for 1 day, and electroporation of "Armed"-T cells prepared in Example 6.1 for 3 days. Wherein the control cells are T cells that are not electroporated and do not comprise GPC3 CAR.

The results in Figure 11 show that the "Armed"-T cells have a similar ability to express CAR as cells expressing only GPC3 CAR.

### 6.2 Tumor killing ability

The mixed cells of D1-T, D2-T and D3-T cells prepared in Example 1.2 and the "Armed"-T cells prepared in Example 6.1 were reapectivley co-incubated with Calcein ⁻AM labeled target cells at different effect-target ratios. The killing of the target cells is detected.

After overnight incubation, flow cytometry was used to detect the degree of target cell killing. The results are shown in Figure 12. Wherein 1-6 respectively represent killing to target cells after: incubation of control cells with target cells for 1 day, incubation of control cells with target cells for 2 days, incubation of mixed cells of D1-T, D2-T and D3-T cells with target cells for 1 day, incubation of control cells with target cells for 2 days, incubation of mixed cells of D1-T, D2-T and D3-T cells with target cells for 2 days, incubation of "Armed" -T cells with target cells for 1 day, and incubation of "Armed" -T cells with target cells for 2 days. The control cells are T cells that are not electroporated and do not comprise GPC3 CAR.

The results in Figure 12 show that "Armed"-T cells have an ability to specifically kill tumor cells *in vitro* similar to unarmed CART.

### 6.3 Tumor killing ability of molecules secreted in the supernatant

The supernatant of the mixed cells of D1-T, D2-T and D3-T cells prepared in Example 1.2 and "Armed"-T cells prepared in Example 6.1 after overnight culture were added to PBMC and Calcein ⁻AM labled incubation system of HepG 2 target cells with different efficiency-to-target ratio. The killing of target cells was detected.

Flow cytometry was used to detect the killing efficiency of target cells. The results are shown in Figure 13. Wherein, 1-4 respectively represent killing to target cells after: incubation of control cells with target cells for 1 day, incubation of control cells with target cells for 2 days, incubation of "Armed"-T cells with target cells for 1 day, and incubation of "Armed"-T cells with target cells for 2 days.

The results in Figure 13 show that the cell supernatant of Armed-T cells can help PBMC kill target cells. Wherein, the killing ability of 2-day incubation is stronger than that of 1-day incubation.

### 6.4 Ability to activate immune cells

The mixed cells of donor 1-T cells, donor 2-T cells and donor 3-T cells prepared in Example 1.1, and the mixed cells of D1-T, D2-T and D3-T cells prepared in Example 1.2 and the "Armed"-T cells prepared in Example 6.1 were respectively incubated overnight with the target cell HepG 2 at an effect-target ratio of 6:1. 100 µL of the cell supernatant collected after incubation was mixed and cultured with 2e5 PBMC cells. After overnight culture, positivity rate and MFI of the CD69 T cells and NK cells in PBMC were detected.

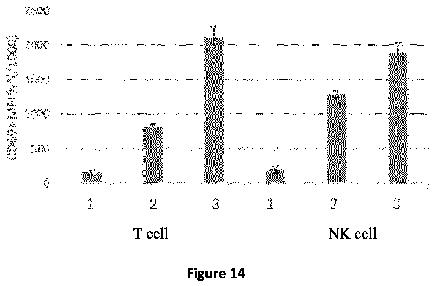

The results are shown in Figure 14. Wherein, 1-3 respectively represent expression of CAR T cells results of mixed cells of donor 1-T cells, donor 2-T cells and donor 3-T cells, mixed cells of D1-T, D2-T and D3-T cells, "Armed"-T cell. The results in Figure 14 illustrate that "Armed"-T cells have a more effective ability to activate T cells and NK cells.

### 6.5 Tumor killing ability and ability to activate immune cells

Nucleic acid molecules (mRNA) encoding CAR and BiTE (corresponding DNA nucleotide sequences are shown in SEQ ID NO. 4) were repectively transfected into T cells that were activated for 5-9 days using Dynal Bead: cells at a 1:1 ratio by electroporation to generate CART and BiTE T. T cells that were not electroporated were used as control cells. The control cells, CART and BiTE T cells were incubated with target cells HepG 2 at an effect-target ratio of 6:1 respectively. Cell supernatant A was collected after incubation.

CART and BiTE T cells were cultured respectively overnight, and the corresponding supernatants B were collected. 50 µL of supernatant B was added to a total of 150 µL of mixed cells of PBMC and target cells with an effect-target ratio of 15:1. After overnight incubation, supernatant D was collected.

150 µL of collected supernatant A and supernatant D were mixed with Calcein-AM labeled 2E5 PBMC in a 96-well plate respectively. After overnight, flow cytometry was used to detect the activation of T and NK cells in Calcein-AM labeled PBMC, which represents the positive rate and the average fluorescence intensity MFI of the molecule CD 69.

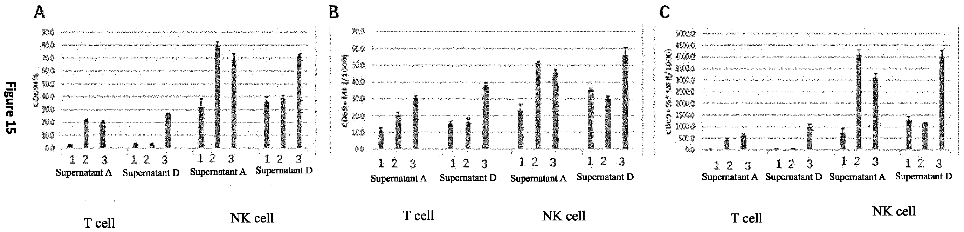

The results are shown in Figures 15A-15C. Figures 14A-14C show the CD69 positivity rate, CD69 MFI and the comprehensive change of CD69 (i.e., the product of the positivity rate and MFI value) respectively. Wherein, 1-3 represent the results of the supernatants corresponding to control cells, CART cells and BiTE-T cells respectively.

The results in Figure 15 illustrate that BiTE T has the ability to kill tumor cells and has a better ability of By-Stand activation of T/NK cells in PBMCs.

### Example 7 Detection of cryopreservation and recovery capabilities

7.1
The prepared GPC3 CAR plasmid (in which the nucleotide sequence encoding GPC3 CAR is shown in SEQ ID NO. 2) was electroporated into human T cells that have been activated with Dynal bead for 2-3 days using CRISPR/sgRNA system to obtain CART cells D1-T, D 2-T, and D 3-T with anti-GPC-3 CAR knocked in.

The CART cells were activated with target cells HepG2, stimulated every 4-7 days, freezed and stored for 4-7 days after stimulation.

After the cryopreserved cells were recovered, the cell viability and proliferation were detected.

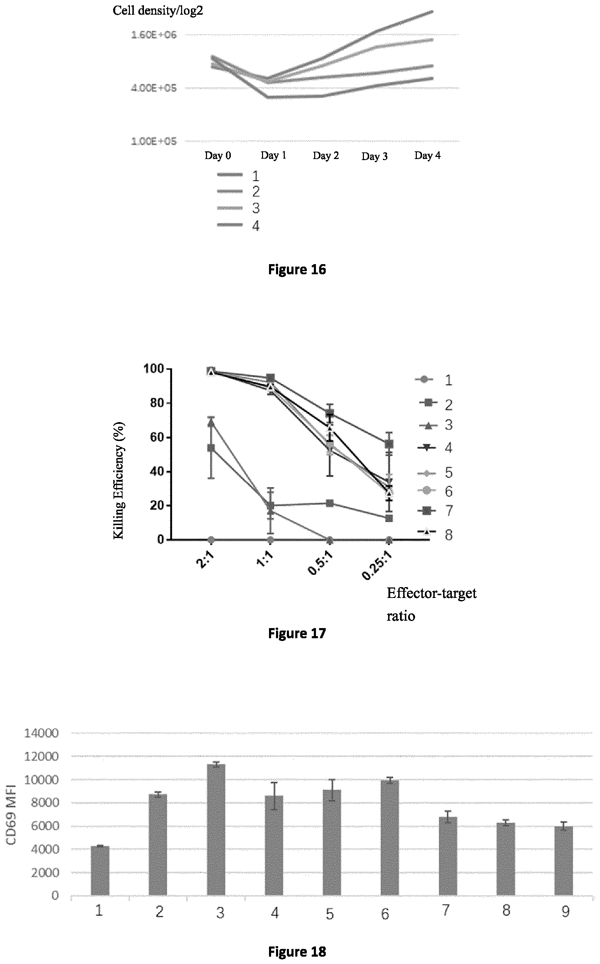

The results are shown in Figure 16. In Figure 16, 1-4 respectively represent: 1: CART cells that are not activated with target cells; 2: CART cells that are activated once with target cells; 3: CART cells that are activated with target cells twice; 4: CART cells activated with target cells 3 times.

The results in Figure 16 illustrate that the allogeneic T cell of the present application may be activated, expanded, revived and/or cryopreserved multiple times.

7.2
CART cells prepared in Example 7.1 were stimulated with the target cell HepG 2 (E:T=2:1), once every 4-7 days, and frozen 4-7 days after stimulation. When detecting cell lethality, effector cells were mixed with target cells according to the effect-target ratio shown in the figure, incubated overnight and then the killing efficiency was detected. Unless otherwise specified, cells were cryopreserved and recovered.

The results are shown in Figure 17. Figure 17 shows the killing efficiency of tumor cells. In Figure 17, 1-8 respectively represent: 1: control cells; 2: CART cells prepared in Example 4.1; 3: CART cells not activated with target cells; 4: CART cells activated once with target cells; 5: CART cells activated twice with target cells; 6: CART cells activated 3 times with target cells; 7: CART cells activated 3 times with target cells (not frozen); 8: CART cells activated 4 times with target cells (not frozen). Wherein, the control cells are T cells that are not electroporated and do not express CAR.

The supernatants from the above groups 1-8 mixed with the target cells and incubated overnight at an effect-target ratio of 2:1 were collected. 2e5 PBMC cells were added 150 µL of the supernatant of each group. After culturing overnight, the mean fluorescence intensity (MFI) of T cells and NK cells was measured by flow cytometry.

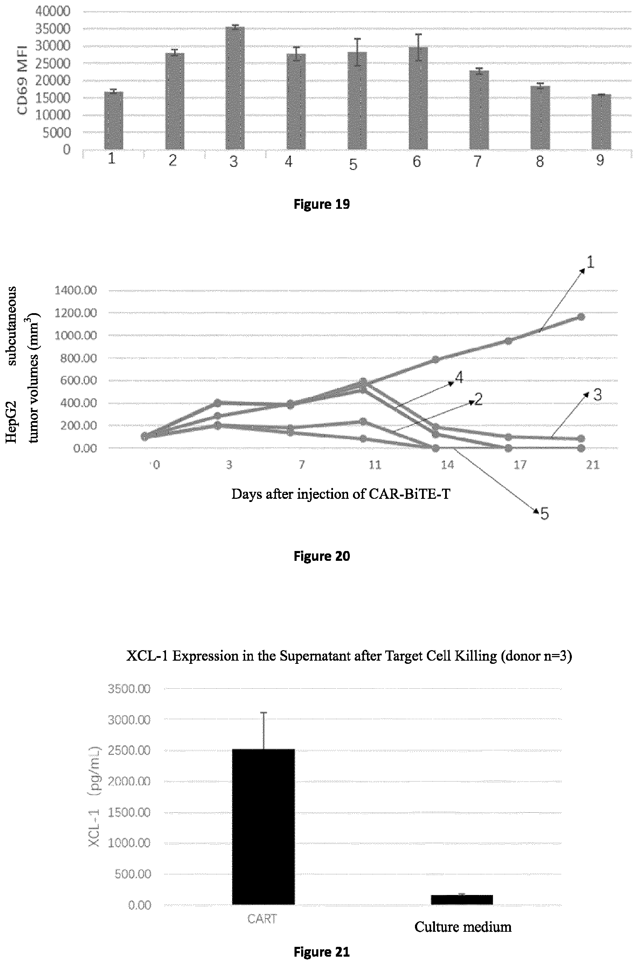

Figures 18 and 19 show the activation effects on T cells and NK cells respectively. Cell activation is achieved by stimulating CART cells with target cell HepG 2 (E:T=2:1). Stimulate every 4-7 days, freeze and store 4-7 days after stimulation. 1-9 in the figure respectively represent the results corresponding to the supernatant after incubation of the following cells with HepG 2 target cells: 1: control cells; 2: CART cells prepared in Example 4.1; 3: positive control (which is a internal positive control sample, that is, the supernatant after a portion of cryopreserved CART was co-incubated with target cells and verified to be able to activate T/NK cells in PBMC); 4: CART cells prepared in Example 7.1 without target cell activation; 5: CART cells prepared in Example 7.1 that were activated once using target cells; 6: CART cells prepared in Example 7.1 that were activated twice using target cells; 7: CART cells prepared in Example 7.1 that were activated three times using target cells; 8: CART cells prepared in Example 7.1 that were activated three times using target cells (not frozen); 9: CART cells prepared in Example 7.1 that were activated four times using target cells (not frozen). Wherein, the control cells are T cells that are not electroporated and do not express CAR.

The results of Figures 17-19 show that the allogeneic CART cells with CAR knock-in of the present application 1) have a strong ability to kill tumors and the ability of By-Stand activation of T/NK cells in PBMC; 2) the ability to activate T/NK cells begins to decrease after activation by target cells 3 times.

### Example 8 Detection of the killing ability of tumors in vivo

### 8.1 Preparation of CAR-Bite T cells

The prepared GPC3 CAR-BITE plasmids (wherein the nucleotide sequence encoding GPC3 CAR-BITE is shown in SEQ. ID NO. 4) were electroporated into human T cells that were activated with Dynal bead for 2-3 days to obtain CAR-BITE cells with knock-in anti-GPC-3CAR-BITE using CRISPR/sgRNA system.

### 8.2 Establishment of animal models and detection of the killing ability of tumors in vivo

NCG mice were subcutaneously loaded with tumor-bearing HepG2 cells. When the tumors grew to 100mm³, they were randomly divided into groups, with 4 mice in each group. Intratumoral injection of 3e6 recovered cells prepared in 8.1.

The ability to kill tumors in the body was tested, and the results are shown in Figure 20. In Figure 20, 1-5 respectively represent, the average tumor growth of 4 mice treated with Mock-CART (anti-CD 19 CAR T cells), growth of tumor volume (mm³) of the first mouse treated with CAR-BiTE T, the second mouse treated with CAR-BiTE T, the third mouse treated with CAR-BiTE T, and the fourth mouse treated with CAR-BiTE T.

The results in Figure 20 illustrate that the allogeneic T cell of the present application can effectively kill tumors *in vivo* and reduce tumor volume through intratumoral injection.

### Example 9 Detection of the ability to activate immune response after killing tumors

### 9.1 Detection of XCL-1 expression in the supernatant after target cell killing

The 3 donor CAR-T cells prepared in Example 1.3 were respectively co-incubated with the target cell Huh-7 overnight, the respective supernatants were collected to detect XCL-1. As shown in Figure 21, in the supernatant of the target cells killed by CART, the expression level of XCL-1 was significantly higher than the level of XCL-1 in the culture medium.

### 9. 2 Detection of the effect of supernatant in recruiting cDC1 cells after killing target cells

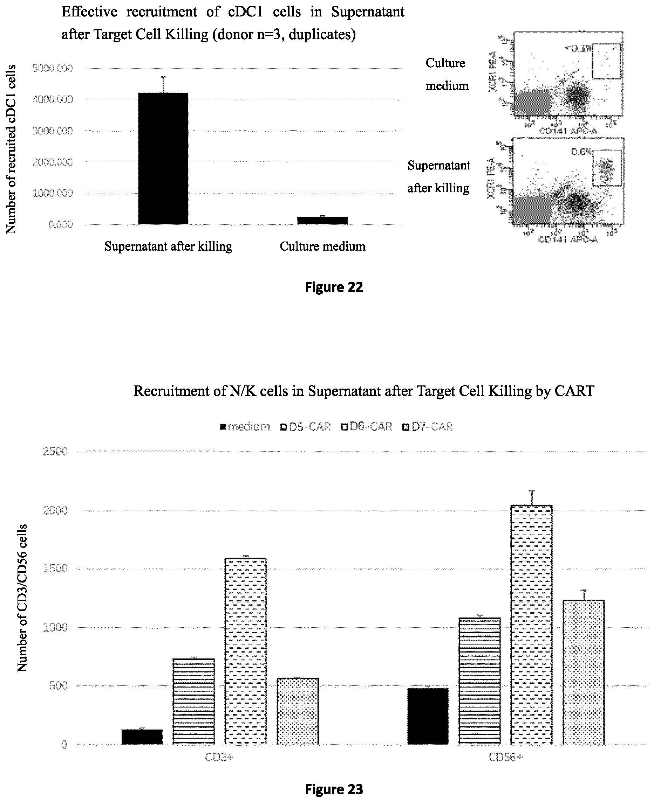

The 3 donor CAR-T cells prepared in Example 1.3 were respectively co-incubated with the target cell Huh-7 overnight, the respective supernatants were collected. 500 µL of supernatant from each group were added to the lower chamber of Tranwell. Fresh PBMCs with CD3 and CD14 removed were labeled with Calcein-AM, and the concentration was adjusted to 1e7/mL. 100 µL was added to the upper chamber of Tranwell, and the number of cDC1 (XCR 1 and CD141 double-positive) cells flowing down from the lower chamber that were subject to chemotaxis were detected after incubation for 1.5 hours. As shown in Figure 22, the supernatant after target cell killing significantly recruited more cDC1 cells.

### 9.3 Detection of the effect of supernatant in recruiting T/NK cells after target cell killing

After the 3 donor CARTs prepared in Example 1.3 were co-incubated with the target cells Huh-7 overnight, the respective supernatants were collected. 500 µL of supernatant from each group was added to the lower chamber of Tranwell. Fresh PBMCs were labeled with Calcein -AM, and the concentration was adjusted to 1e7/mL. 100 µL was added to the upper chamber of Tranwell and the number of T/NK cells flowing down from the lower chamber that were subject to chemotaxis were detected after incubation for 1.5 hours. As shown in Figure 23, the supernatant after each donor CAR-T cell kills target cells can significantly recruit more T/NK cells.

### 9.4 Detection of activation function of T/NK cells by supernatant after target cell killing

After the 3 donor CAR-T cells prepared in Example 1.3 were respectively co-incubated with the target cell Huh-7 and the irrelevant target cell SK-Hep1 overnight, the respective supernatants were collected. 2e5 Calcein -AM labeled PBMC cells were added to 100 µL of the supernatant of each group, and after culturing overnight, the CD69 mean fluorescence intensity (MFI) of T cells and NK cells in PBMCs was measured by flow cytometry.

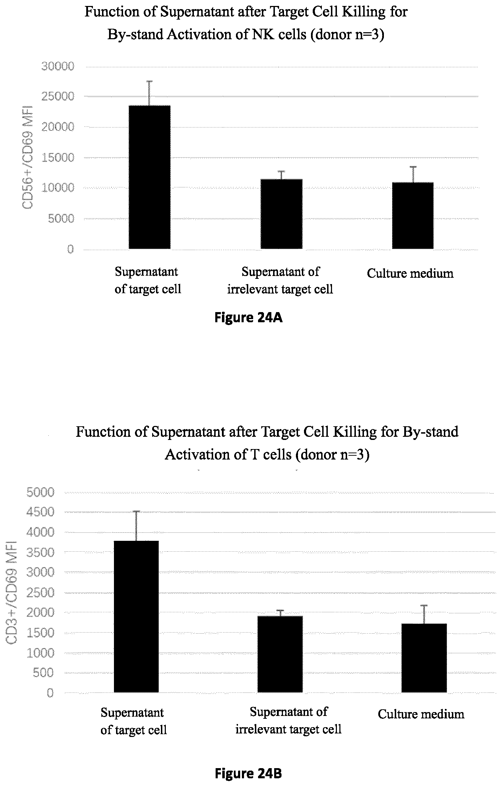

The results are shown in Figures 24A and 24B. The supernatant incubated with the target cell Huh-7 increased the CD69 MFI of CD3 T cells and CD56 NK cells.

### 9.5 Detection of the activation effect of supernatant after target cell killing on immune cells in PBMCs

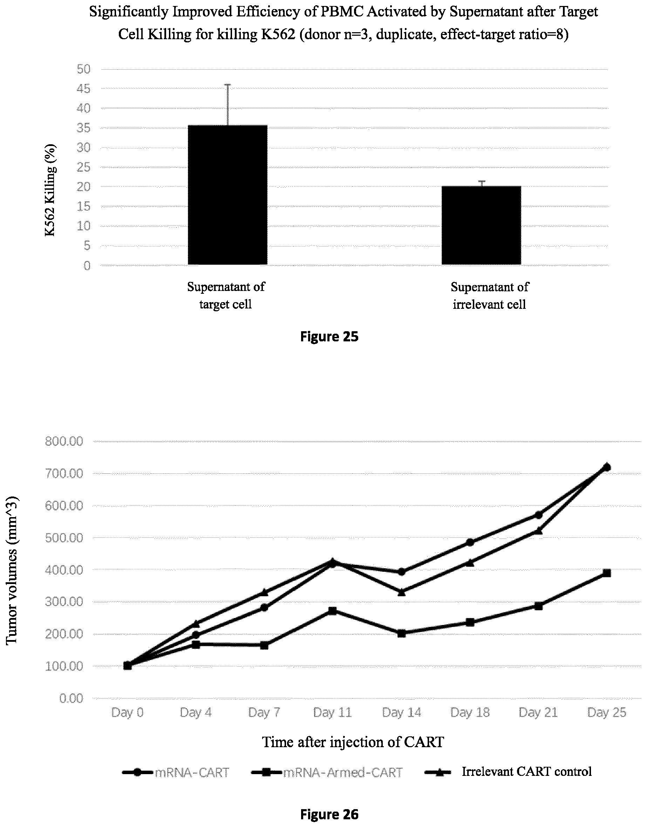

The 3 donor CAR-T cells prepared in Example 1.3 were respectively co-incubated with the target cell Huh-7 and the unrelated target cell SK-Hep1 overnight, and the respective supernatants were collected. After incubating the supernatants with PBMCs overnight, the collected PBMCs were incubated with K562 to verify whether PBMCs were activated in the collected supernatants. As shown in Figure 25, the killing effect of the supernatant of CART and Huh-7 on K562 was significantly higher than that of the supernatant of CAR-T cells incubated with the unrelated target cell SK-Hep1. It shows that the supernatant after killing the target cells can activate immune cells in PBMCs.

### Example 10 Detection of the killing ability of tumors in vivo

10.1
GPC 3 mRNA-CAR prepared in Example 1.2 and the GPC 3 mRNA-Armed CAR that simultaneously expresses IL-12 and IL-1b (wherein the nucleotide sequence encoding GPC3 CAR is shown in SEQ ID NO. 2) were electroporated into Human T cells that were activated with Dynal bead to obtain knock-in anti-GPC-3 mRNA-CART and GPC 3 mRNA-Armed - CART cells.

When the subcutaneous tumor-bearing HepG2 cell tumors of NCG mice grew to 100 mm³, they were randomly divided into groups with 4 mice in each group. A total of 4 injections of 3e6 recovered GPC-3 mRNA-CART and GPC 3 mRNA-Armed-CART cells by intratumoral injections twice a week.

The nucleotide sequence of the nucleic acid molecule encoding the cytokine IL12a is shown in SEQ ID NO. 8.

The nucleotide sequence of the nucleic acid molecule encoding the cytokine IL12b is shown in SEQ ID NO. 9.

The nucleotide sequence of the nucleic acid molecule encoding the cytokine IL1b is shown in SEQ ID NO. 5.

The results are shown in Figure 26, which shows the average tumor volume of four mice. The results showed that after injection of unrelated CART control (anti-CD19 CAR T cells), GPC-3-mRNA-CART and GPC 3 mRNA-Armed-CART, the GPC 3 mRNA-Armed-CART group obviously had a tumor-inhibitory effect on tumor growth.

10.2
The GPC 3 CAR-2A-IL15-2A-IL21 plasmids prepared in Example 1.3 were electroporated into human T cells that were activated with Dynal bead for 2-3 days using the CRISPR/sgRNA system to obtain knock-in anti-GPC-3 CART cells. Anti-GPC-3 CART cells are fresh cells maintained by activation and expansion of target cells Huh-7 at the effect-target ratio E:T=2:1. Wherein, CAR targets GPC3, and the nucleotide sequence of the nucleic acid molecule encoding GPC 3 CAR-2A-IL15-2A-IL21 is shown in SEQ ID NO: 11.

When the subcutaneous tumor-bearing HepG2 cell tumors of Nude mice grew to 100mm³, they were randomly divided into CART group, solvent control group, and extended injection CART group. In the CART group, CART cells were injected once within 7 days into the tumor, for a total of 5 times. In the extended injection CART group, injection was started after the tumor reached a volume of 700 mm³, once a week, for a total of 2 injections.

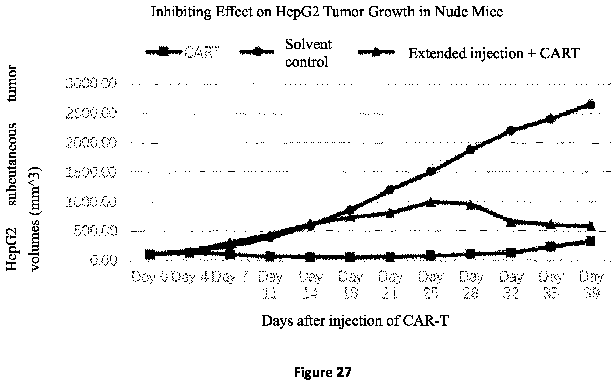

The results are shown in Figures 27. The statistics are the average tumor growth of the four experimental mice. The results show that compared with Nude mice with residual immune function, the allogeneic CART cells of the present application can still effectively kill tumors in vivo and reduce the tumor volume through multiple intratumoral injections, though the survival time in mice is greatly reduced compared with NCG mice. It is expected that it will also provide neoantigens released after killing the tumor.

The above detailed description is provided for explanation and example purposes and is not intended to limit the scope of the appended claims. Variations of the embodiments listed in the present application are apparent to those skilled in the art and are reserved within the scope of the appended claims and their equivalents.

## Claims

1. A T cell product for the administration to a subject, wherein the product comprises at least one allogeneic T cell that expresses at least one MHC molecule recognized as being exogenous by at least one T cell of the subject, and an alloreaction is caused after recognization of the MHC molecule by at least one TCR of the subject.

2. The T cell product according to claim 1, wherein the allogeneic T cell is not subjected to an operation for reducing or eliminating immune rejection.

3. The T cell product according to claim 2, wherein the operation for reducing or eliminating immune rejection comprises knocking out CD52 gene of an allogeneic T cell; and/or changing HLA-I molecule of an allogeneic T cell.

4. The T cell product according to any one of claims 1-3, wherein in the allogeneic T cell, a molecule capable of causing an alloreaction in a subject is not modified.

5. The T cell product according to claim 4, wherein the molecule capable of causing an alloreaction in a subject comprises a MHC molecule of the allogeneic T cell.

6. The T cell product according to any one of claims 1-5, wherein the subject comprises a patient with a tumor.

7. The T cell product of claim 6, wherein the tumor comprises a solid tumor and/or non-solid tumor.

8. The T cell product according to any one of claims 1-7, wherein the allogeneic T cell is derived from a source different from the subject.

9. The T cell product according to any one of claims 1-8, wherein the allogeneic T cell is derived from two or more donors that are different from the subject.

10. The T cell product according to any one of claims 1-9, wherein the allogeneic T cell expresses a MHC molecule that is recognized as being exogenous by at least one T cell of the subject.

11. The T cell product according to any one of claims 1-10, wherein the allogeneic T cell expresses two or more MHC molecules that are recognized as being exogenous by at least one T cell of the subject.

12. The T cell product according to any one of claims 1-11, wherein the MHC molecule comprises MHC I and/or MHC II.

13. The T cell product according to any one of claims 1-12, wherein the allogeneic T cell comprises helper T cell (Th) and/or killer T cell (CTL).

14. The T cell product according to any one of claims 1-13, wherein the recognition of the MHC molecule by at least one TCR of the subject promotes a TH-1 immune response in the subject.

15. The T cell product according to any one of claims 1-14, wherein the product has the ability to reduce the risk of causing graft-versus-host disease (GVHD) in the subject by the allogeneic T cell.

16. The T cell product according to any one of claims 1-15, wherein compared with an allogeneic T cell without an engineering modification, the allogeneic T cell with an engineering modification reduces the risk of graft-versus-host disease (GVHD) in the subject by the allogeneic T cell.

17. The T cell product according to claim 16, wherein the engineering modification comprises the following step: down-regulating the expression and/or activity of a CD3-related gene, TRAC gene and/or TRBC gene in the allogeneic T cell.

18. The T cell product according to any one of claims 16-17, wherein compared with the allogeneic T cell without an engineering modification, the expression and/or activity of a CD3-related gene, TRAC gene and/or TRBC gene is downregulated in the allogeneic T cell with an engineering modification.

19. The T cell product according to any one of claims 15-17, wherein the engineering modification comprises gene mutation and/or gene silencing.

20. The T cell product according to any one of claims 16 to 19, wherein the engineering modification comprises administering to the allogeneic T cell one or more substances selected from the group consisting of an antisense RNA, siRNA, shRNA, CRISPR/Cas system, RNA editing system, RNA-guided endonuclease, zinc finger protease, Mega-TAL nuclease, TALENs, Sleeping beauty transposon system and Meganucleases.

21. The T cell product according to any one of claims 16-20, wherein the engineering modification comprises administering a CRISPR/Cas system to the allogeneic T cell.

22. The T cell product according to any one of claims 16-21, wherein the engineering modification comprises administering to the allogeneic T cell a sgRNA targeting the exon region of the CD3-related gene, TRAC gene and/or TRBC gene.

23. The T cell product according to any one of claims 1-22, wherein at least one of the allogeneic T cell with modification expresses an antibody or antigen-binding fragment thereof, a chimeric antigen receptor (CAR), a cytokine and/or a chemokine.

24. The T cell product according to any one of claims 1-23, wherein at least one of the allogeneic T cell with modification comprises a nucleic acid encoding an antibody or antigen-binding fragment thereof, a chimeric antigen receptor (CAR), a cytokine, and/or chemokine.

25. The T cell product of claim 24, wherein the modification comprises the transfection of the nucleic acid into the allogeneic T cell.

26. The T cell product of claim 25, wherein the transfection comprises the viral transfection and/or non-viral transfection.

27. The T cell product of claim 26, wherein a plasmid is used for the non-viral transfection.

28. The T cell product according to claim 27, wherein the plasmid is a circular plasmid or a supercoiled plasmid.

29. The T cell product according to any one of claims 27-28, wherein the plasmid comprises a nucleic acid encoding an antibody or an antigen-binding fragment thereof, a chimeric antigen receptor (CAR), a cytokine and/or a chemokine.

30. The T cell product according to any one of claims 24-29, wherein the nucleic acid comprises a ssDNA and/or dsDNA.

31. The T cell product according to any one of claims 27-30, wherein the plasmid comprises a plasmid produced by bacteria.

32. The T cell product according to any one of claims 27-31, wherein the plasmid comprises a Nano plasmid and/or minicircle DNA vector.

33. The T cell product according to any one of claims 25-32, wherein the transfection comprises the stable transfection and/or transient transfection.

34. The T cell product according to claim 33, wherein the transient transfection comprises the electroporation.

35. The T cell product according to any one of claims 33-34, wherein the transient transfection comprises the mRNA transfection.

36. The T cell product according to any one of claims 33-35, wherein the stable transfection comprises the knock-in by using a transposon system and/or using gene editing method.

37. The method according to claim 36, wherein the gene editing method is selected from the group consisting of: a CRISPR/Cas system, RNA editing system ADAR, RNA-guided endonucleases, zinc finger nucleases, Mega-TAL nucleases, TALENs, and Meganucleases.

38. The T cell product according to any one of claims 36-37, wherein the transposon system comprises a Sleeping beauty transposon system.

39. The method according to any one of claims 27-38, wherein the plasmid is used as a donor plasmid in a knock-in process via gene editing.

40. The T cell product according to any one of claims 27-39, wherein in a transfection mixture comprising the plasmid, the content of a nucleic acid molecule or fragment thereof derived from a microbial genome is less than about 10% (w/w) of the total content of nucleic acid molecules in the transfection mixture.

41. The T cell product according to claim 40, wherein in the transfection mixture comprising the plasmid, the content of a nucleic acid molecule or fragment thereof derived from a microbial genome is less than about 2% (w/w) of the total content of nucleic acid molecules in the transfection mixture.

42. The T cell product according to any one of claims 40-41, wherein in the transfection mixture comprising the plasmid, the content of a nucleic acid molecule or fragment thereof derived from a microbial genome is less than about 5‰ (w/w)of the total content of nucleic acid molecules in the transfection mixture.

43. The T cell product according to any one of claims 40-42, wherein in the transfection mixture comprising the plasmid, the content of a nucleic acid molecule or fragment thereof derived from a microbial genome is less than about 1‰ (w/w) of the total content of nucleic acid molecules in the transfection mixture.

44. The T cell product according to any one of claims 27-43, wherein in the transfection mixture comprising the plasmid, the content of a nucleic acid molecule or fragment thereof with a size of at least about 48 kb is less than about 10% (w/w) of the total content of nucleic acid molecules in the transfection mixture.

45. The T cell product according to claim 44, wherein in the transfection mixture comprising the plasmid, the content of a nucleic acid molecule or fragment thereof with a size of at least about 48 kb is less than about 2% (w/w) of the total content of nucleic acid molecules in the transfection mixture.

46. The T cell product according to any one of claims 44-45, wherein in the transfection mixture comprising the plasmid, the content of a nucleic acid molecule or fragment thereof with a size of at least about 48 kb is less than about 5‰ (w/w) of the total content of nucleic acid molecules in the transfection mixture.

47. The T cell product according to any one of claims 44-46, wherein in the transfection mixture comprising the plasmid, the content of a nucleic acid molecule or fragment thereof with a size of at least about 48 kb is less than about 1‰ (w/w) of the total content of nucleic acid molecules in the transfection mixture.

48. The T cell product according to any one of claims 44-47, wherein the size of the nucleic acid molecule or a fragment thereof with a size of at least about 48 kb is at least about 1 Mb.

49. The T cell product according to any one of claims 44-48, wherein the size of the nucleic acid molecule or a fragment thereof with a size of at least about 48 kb is at least about 10 Mb.

50. The T cell product according to any one of claims 44-49, wherein the nucleic acid molecule or a fragment thereof with a size of at least about 48 kb is derived from a microorganism.

51. The T cell product according to claim 50, wherein the microorganism is selected from the group consisting of: bacteria, fungi, actinomycetes, mycoplasmas, chlamydiae, rickettsiae, and spirochetes.

52. The T cell product according to any one of claims 50-51, wherein the microorganism comprises Gram-negative bacteria.

53. The T cell product according to any one of claims 50-52, wherein the microorganism comprises *Escherichia coli.*

54. The T cell product according to any one of claims 27-53, wherein the plasmid is treated with deoxyribonuclease (DNase).

55. T cell product according to claim 54, wherein the DNase is capable of non-specifically cleaving linear DNA.

56. T cell product according to any one of claims 54-55, wherein the DNase is an exonuclease.

57. The method according to any one of claims 54-56, wherein the treatment comprises contacting the transfection mixture with the DNase in the presence of Mg²⁺ and Ca²⁺.

58. T cell product according to according to claim 57, wherein after the treatment by the DNase, the content of a nucleic acid molecule or fragment thereof derived from a microbial genome in the transfection mixture comprising the plasmid is less than about 10% (w/w) of the total content of nucleic acid molecules in the transfection mixture.

59. T cell product according to according to claim 57, whererin after the treatment by the DNase, the content of a nucleic acid molecule or fragment thereof with a size of at least about 48 kb in the transfection mixture comprising the plasmid is less than about 10% (w/w) of the total content of nucleic acid molecules in the transfection mixture.

60. The T cell product according to any one of claims 23-59, wherein the antibody or an antigen-binding fragment thereof comprises a bispecific antibody.

61. The T cell product according to any one of claims 23-60, wherein the antibody or an antigen-binding fragment thereof comprises a bispecific T cell engager (BiTE).

62. The T cell product according to any one of claims 23-61, wherein the CAR comprises an antigen-binding domain, hinge region, transmembrane domain, costimulatory domain and signaling domain.

63. The T cell product according to claim 62, wherein the antigen binding domain specifically binds a tumor associated antigen (TAA).

64. The T cell product according to claim 63, wherein the antigen-binding domain specifically binds one or more tumor-associated antigens.

65. The T cell product according to any one claims 62-64, wherein the antigen binding domain specifically binds GPC3 and CD73.

66. The T cell product according to any one of claims 62-65, wherein the antigen binding domain specifically binds GPC3 and CD147.

67. The T cell product according to any one of claims 62-66, wherein the antigen-binding domain is selected from the group consisting of: a monoclonal antibody, polyclonal antibody, human antibody, humanized antibody, single domain antibody, nano antibody, and antigen-binding fragment thereof.

68. The T cell product according to any one of claims 62-67, wherein the hinge region comprises a polypeptide from a protein selected from the group consisting of CD28, HLA-A, HLA-B, HLA-C, HLA-G, HLA-DQ, HLA-DP, HLA-DR, CD8, Fc, IgG, IgD, 4-1BB, CD4, CD27, CD7 and PD1.

69. The T cell product according to any one of claims 62-68, wherein the transmembrane domain comprises a polypeptide from a protein selected from the group consisting of: CD27, CD7, PD1, TRAC, TRBC, CD28, CD3e, CD45, CD4, CD5, CD8, CD9, CD16, CD22, CD33, CD37, CD64, CD80, CD86, CD134, CD137 and CD154.

70. The T cell product according to any one of claims 62-69, wherein the costimulatory domain comprises a polypeptide from a protein selected from the group consisting of CD28, CD137, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, and ligand of CD83.

71. The T cell product according to any one of claims 62-70, wherein the signaling domain comprises a polypeptide from a protein selected from the group consisting of CD3ζ, FcRγ (FCER1G), FcγRIIa, FcRβ (FcεR1b), CD3γ, CD3δ, CD3ε, CD79a, CD79b, DAP10, DAP12, Igα receptor, Igβ receptor, BLV gp30 (bovine leukemia virus gp30), EBV (Ep-stein-Barr virus) LMP2A, Simian Immunodeficiency Virus PBj14 Nef and the immunoreceptor tyrosine activation motif (ITAM).

72. The T cell product according to any one of claims 23-71, wherein the cytokine is selected from the group consisting of: IL-1, IL-7, IL-12, IL-13, IL-15, IL-16, IL-17A, IL-17F, IL - 18, IL-21, IL-23, TNFα, CXCL1, CD38, CD40, CD69, IgG, IP-10, L-17A, MCP-1 and PGE2.

73. The T cell product according to any one of claims 23-72, wherein the chemokine is selected from the group consisting of: CCL1-CCL28 and CXCL1-CXCL17.

74. The T cell product according to any one of claims 24-73, wherein after transfection, at least one of the allogeneic T cell comprises at least one plasmid comprising the nucleic acid.

75. The T cell product according to any one of claims 61-74, wherein at least one of the allogeneic T cell expresses a chimeric antigen receptor (CAR), or expresses a bispecific T cell adapter (BiTE).

76. The T cell product according to any one of claims 1-75, wherein at least one of the allogeneic T cell expresses two or more proteins.

77. The T cell product according to any one of claims 23-76, wherein at least two of the allogeneic T cells expressing different chimeric antigen receptor (CAR) and/or cytokine are mixed proportionally.

78. The T cell product according to any one of claims 1-77, further comprising an autologous T cell of the subject.

79. The T cell product according to any one of claims 1-78, further comprising one or more other modified T cells that have a reduced ability to cause an alloreaction in the body compared with the corresponding unmodified T cells.

80. The T cell product according to any one of claims 1-79, wherein the other modified T cells have a reduced expression and/or activity of MHC molecules compared with corresponding unmodified T cells.

81. A pharmaceutical composition comprising the T cell product according to any one of claims 1-80 and a pharmaceutically acceptable excipient.

82. The pharmaceutical composition according to claim 81, wherein the excipient is suitable for injection.

83. The pharmaceutical composition according to any one of claims 81-82, wherein the excipient is suitable for intratumoral injection.

84. A kit comprising the T cell product according to any one of claims 1-80 and an injection equipment.

85. Use of the T cell product according to any one of claims 1-80, the pharmaceutical composition according to any one of claims 81-83 and/or the kit according to claim 84 in the manufacture of a medicament for the prevention, alleviation, and/or treatment of a tumor.

86. The use according to claim 85, wherein the tumor comprises a solid tumor.

87. The use according to any one of claims 85-86, wherein the medicament is formulated to be suitable for injection.

88. The use according to any one of claims 85-87, wherein the medicament is formulated to be suitable for intratumoral injection.

89. A method for improving tumor microenvironment, comprising the following step: administering to a subject the T cell product according to any one of claims 1-80, the pharmaceutical composition according to any one of claims 81-83, and/or the kit according to claim 84.

90. A method for preventing, alleviating, and treating a tumor, comprising the following step: administering to a subject the T cell product according to any one of claims 1-80, the pharmaceutical composition according to any one of claims 81-83, and/or the kit according to claim 84.

91. The method according to claim 90, wherein the administration comprises injection.

92. The method according to any one of claims 90-91, wherein the administration comprises intratumoral injection.

93. The method according to any one of claims 90-92, wherein the administration comprises the intratumoral injection for one or more times.

94. The T cell product according to any one of claims 1-80, the pharmaceutical composition according to any one of claims 81-83, and/or the kit according to claim 84 for use in the prevention, alleviation and treatment of a tumor.
